# EUROPEAN PATENT SPECIFICATION

(11) **EP 1 730 108 B1**
(45) Date of publication and mention of the grant of the patent: **26.03.2008**
(21) Application number: 05706821.5
(22) Date of filing: 10.03.2005
(51) Int. Cl.: C07C 401/00, C07D 333/72

(54) **STEREOSELECTIVE SYNTHESIS OF VITAMIN D ANALOGUES**
STEREOSELEKTIVE SYNTHESE VON VITAMIN-D-ANALOGA
SYNTHESE STEREOSELECTIVE D'ANALOGUES DE VITAMINE D

(30) Priority: 18.03.2004 US 553962 P; 22.03.2004 DK 200400454
(43) Date of publication of application: 13.12.2006
(73) Proprietor: LEO PHARMA A/S, 2750 Ballerup (DK)
(72) Inventor: SABROE, Thomas Peter, DK-2830 Virum (DK); CALVERLEY, Martin, John, DK-2730 Herlev (DK)
(86) International application number: PCT/DK2005/000161
(87) International publication number: WO 2005/087719

(56) References cited:
- WO-A-87/00834
- US-B1- 6 531 460

## Description

The present invention relates to methods of producing calcipotriol {(5Z, 7E, 22E, 24S)-24-cyclopropyl-9,10-secochola-5,7,10(19),22-tetraene-1α-3β-24-triol} or calcipotriol monohydrate by stereoselective reduction. The present invention further provides novel intermediates and methods for the synthesis of the intermediates useful for producing calcipotriol or calcipotriol monohydrate.

### BACKGROUND OF THE INVENTION

Calcipotriol or calcipotriene (structure I) [CAS 112965-21-6] shows a strong activity in inhibiting undesirable proliferation of epidermal keratinocytes [F.A.C.M. Castelijins, M.J. Gerritsen, I.M.J.J. van Vlijmen-Willems, P.J. van Erp, P.C.M. van de Kerkhof; Acta Derm. Venereol. 79, 11, 1999]. The efficiency of calcipotriol and calcipotriol monohydrate (II) in the treatment of psoriasis was shown in a number of clinical trials [D.M. Ashcroft et al.; Brit. Med. J. 320, 963-67, 2000] and calcipotriol is currently used in several commercial drug formulations.

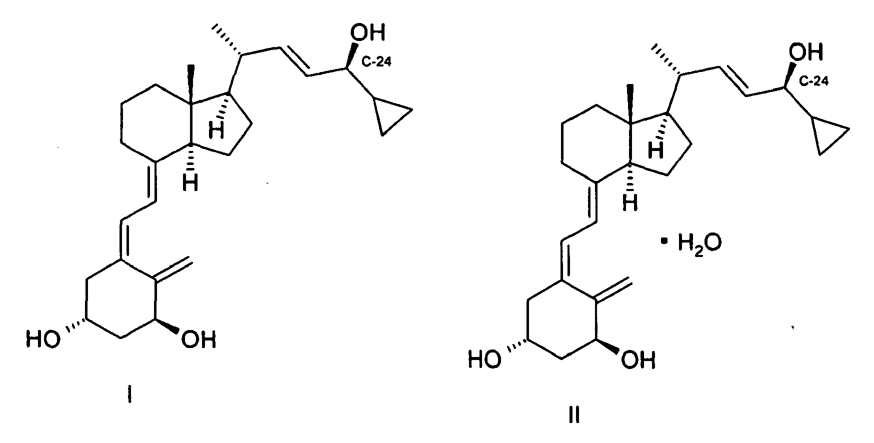

In the preparation of calcipotriol, the specific stereochemistry for the hydroxyl group at C-24 is necessary for full expression of the biological activity. Under current methodology, the required stereochemistry is introduced by one of the following methods:
(i) non-diastereoselective reduction of C-24 keto-trienes followed by the separation of diastereomeric mixtures of the C-24 hydroxyl epimers obtained via chromatography (WO 87/00834 & M.J. Calverley; Tetrahedron, 43 (20), 4609-19, 1987);
(ii) attachment of an enantiopure C-24-hydroxyl-carrying side chain to the vitamin D skeleton (M.J. Calverley, Synlett, 157-59, 1990);
(iii) selective enzymatic esterification of one of C-24 hydroxyl epimers followed by chromatographic separation (WO 03/060094).

The non-diastereoselective reduction of C-24 keto-trienes followed by chromatographic separation of the epimeric mixture (i) is the most widely practiced procedure for obtaining the desired epimer. This reduction process yields mainly the undesired C-24 epimeric alcohol (typically about 60% of the unwanted 24-R epimer) and it is difficult to separate the desired S-epimer from such a mixture by chromatography on a production scale.

The stereoselective synthesis (ii) is still an unfavourable process for scale up due to its multi step nature and cost and due to the fact that toxic intermediates are used.

The enzymatic esterification process (iii) has the disadvantage, apart from the high cost of the enzymes employed, that it introduces, depending on the selectivity of the enzyme, 1-2 additional reaction steps which adds even further costs to the process.

The stereoselective reduction of C-24 ketones directly to the desired C-24 hydroxyl epimers has for example been described for cholesterol derivatives in WO 98/24800 and by M. Ishiguro et al., J. C. S. Chem. Comm., 115-117, 1981. The stereoselective reduction of a side chain triple bond analogue of calcipotriol with unprotected triene system using S-alpine borane has been described by by M. J. Calverly et al. in Bioorg. Med. Chem. Lett., 1841-1844, 3(9), 1993.

A major technical problem of using stereoselective reduction methods for the synthesis of calcipotriol stems from the fact that the unsaturated triene system of hitherto known intermediates for the synthesis of calcipotriol are chemically labile, such as towards Lewis acidic conditions, that they are relatively easily oxidised, and that they are usually not compatible with the typical reduction reaction conditions employed. This results in reduced yields, impure products and tedious work-up procedures, especially on large-scale.

It is an object of this invention to provide an alternative process for the synthesis of calcipotriol, which may overcome one or more of the various problems and disadvantages described above.

The present invention provides a novel process to produce diastereomerically enriched C-24 hydroxyl epimers of calcipotriol derivatives using a novel synthetic pathway comprising a stereoselective reduction step. The present invention further provides novel chemically more stable intermediates where the labile triene system is protected as sulphur dioxide adduct. By producing diastereomerically enriched C-24 hydroxyl epimers of calcipotriol derivatives the yield and the efficacy of the subsequent separation of the desired C-24 S-hydroxyl epimer may be greatly improved.

### SUMMARY OF THE INVENTION

It has surprisingly been found that compounds of general structure III, 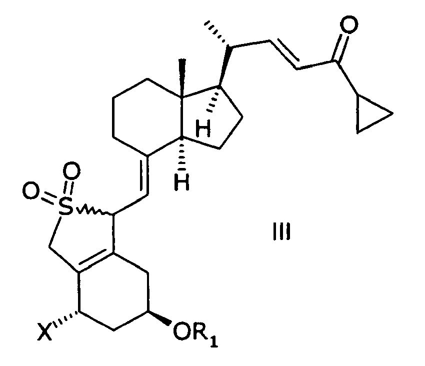 wherein X represents either hydrogen or OR₂,
and wherein R₁ and R₂ may be the same or different and represent hydrogen, or a hydroxy protecting group,
in an inert solvent with a chiral reducing agent or with a reducing agent in the presence of a chiral auxiliary,
to give a mixture of compounds of general structure IVa and IVb, 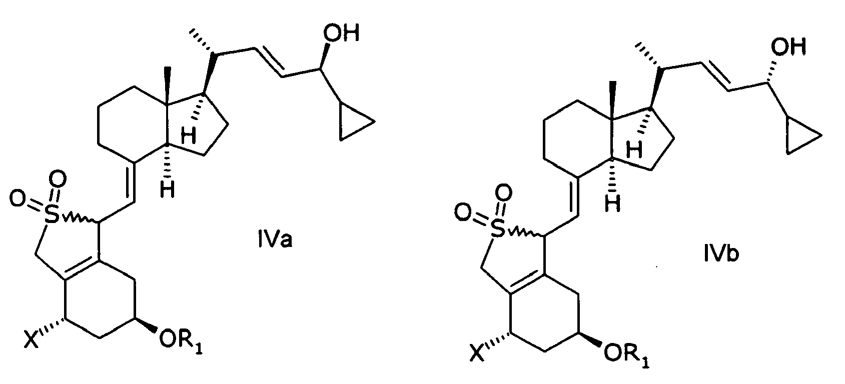 which is enriched with IVa, wherein X, R₁, and R₂ are as defined above.

In a first aspect, this invention relates to a method for producing calcipotriol {(5Z, 7E, 22E, 24S)-24-cyclopropyl-9,10-secochola-5,7,10(19),22-tetraene-1α-3β-24-triol} or calcipotriol monohydrate comprising the steps of:
(a) reducing a compound of general structure III,
   wherein X represents OR₂,
   and wherein R₁ and R₂ may be the same or different and represent hydrogen or a hydroxy protecting group,
   in an inert solvent with a chiral reducing agent or with a reducing agent in the presence of a chiral auxiliary,
   to give a mixture of compounds of general structure IVa and IVb,
   which is enriched with IVa,
   wherein X, R₁ and R₂ are as defined above;
(b) reacting the mixture of compounds of general structure IVa and IVb, which is enriched with IVa, in the presence of a base to give a mixture of compounds of general structure Va and Vb, which is enriched with Va, 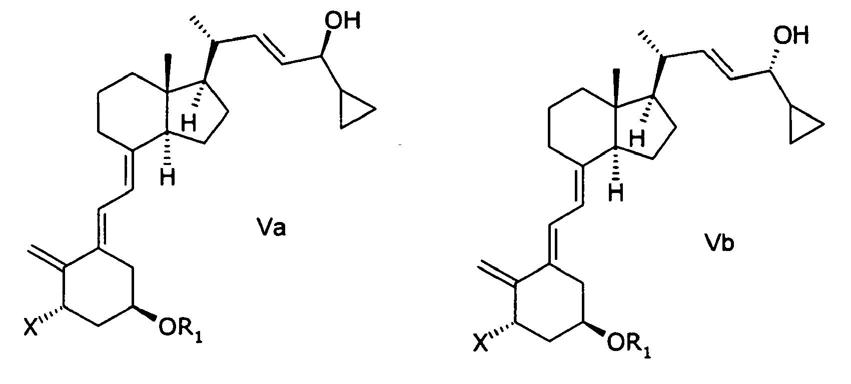 wherein X, R₁ and R₂ are as defined above;
(c) separating the compound of general structure Va from the mixture of compounds of general structure Va and Vb which is enriched with Va, wherein X, R₁ and R₂ are as defined above;
(d) isomerising the compound of general structure Va to the compound of general structu re VIa, 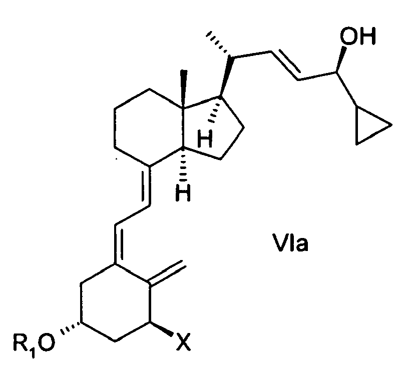 wherein X, R₁ and R₂ are as defined above; and
(e) when R₁ and/or R₂ are not hydrogen, removing the hydroxy protecting group(s) R₁ and/or R₂ of the compound of general structure VIa to generate calcipotriol or calcipotriol monohydrate.
   In a further aspect, this invention relates to a method for producing calcipotriol or calcipotriol monohydrate comprising steps (a) - (b) above and further comprising the steps of:
(f) isomerising the mixture of compounds of general structure Va and Vb, wherein X, R₁ and R₂ are as defined in claim 2, which is enriched with Va, to a mixture of compounds of general structure VIa and VIb, which is enriched with VIa, 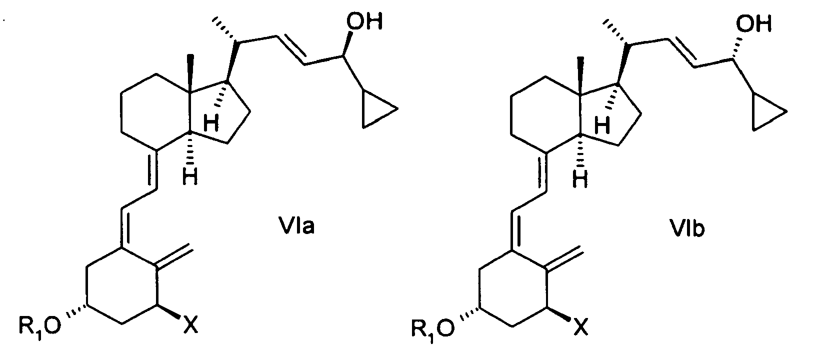 wherein X, R₁ and R₂ are as defined above;
(g) separating the compound of general structure VIa from the mixture of compounds of general structure VIa and VIb which is enriched with VIa, wherein X, R₁ and R₂ are as defined above;
(h) when R₁ and/or R₂ are not hydrogen, removing the hydroxy protecting group(s) R₁ and/or R₂ of the compound of general structure VIa to generate calcipotriol or calcipotriol monohydrate.
   In a still further aspect, this invention relates to a method for producing calcipotriol {(5Z, 7E, 22E, 24S)-24-cyclopropyl-9,10-secochola-5,7,10(19),22-tetraene-1α-3β-24-triol} or calcipotriol monohydrate comprising the steps of:
(j) reducing a compound of general structure III,
   wherein X represents hydrogen,
   and wherein R₁ represents hydrogen or a hydroxy protecting group,
   in an inert solvent with a chiral reducing agent or with a reducing agent in the presence of a chiral auxiliary,
   to give a mixture of compounds of general structure IVa and IVb,
   which is enriched with IVa,
   wherein X and R₁ are as defined above;
(k) reacting the mixture of compounds of general structure IVa and IVb, which is enriched with IVa, in the presence of a base to give a mixture of compounds of general structure Va and Vb, which is enriched with Va,
   wherein X and R₁ are as defined above;
(l) separating the compound of general structure Va from the mixture of compounds of general structure Va and Vb which is enriched with Va, wherein X and R₁ are as defined above;
(m) hydroxylating the compound of general structure Va with a suitable hydroxylating agent, wherein X and R₁ are as defined above to give a compound of general structure Va, wherein X represents OR₂ and R₂ represents hydrogen, and wherein R₁ is as defined above;
(o) isomerising the compound of general structure Va to the compound of general structure VIa,
   wherein X, R₁ and R₂ are as defined above; and
(p) when R₁ is not hydrogen, removing the hydroxy protecting group R₁ of the compound of general structure VIa to generate calcipotriol or calcipotriol monohydrate.
   In a still further aspect, this invention relates to a method for producing calcipotriol or calcipotriol monohydrate comprising steps (j) - (I) of claim 4 and further comprising the steps of:
(q) protecting the C-24 hydroxy group of the compound of general structure Va,
   wherein X represents hydrogen, and wherein R₁ represents hydrogen or a hydroxy protecting group, with a hydroxy protecting group;
(r) hydroxylating the C-24 hydroxy protected compound of general structure Va with a suitable hydroxylating agent, wherein X and R₁ are as defined above to give a C-24 hydroxy protected compound of general structure Va, wherein X represents OR₂ and R₂ represents hydrogen, and wherein R₁ is as defined above;
(s) removing the C-24 hydroxy protecting group of the compound of general structure Va;
(t) isomerising the compound of general structure Va to the compound of general structure VIa,
   wherein X, R₁ and R₂ are as defined above; and
(u) when R₁ is not hydrogen, removing the hydroxy protecting group R₁ of the compound of general structure VIa to generate calcipotriol or calcipotriol monohydrate.

In a still further aspect, this invention relates to a method for producing a compound of general structure III,
wherein X represents either hydrogen or OR₂,
and wherein R₁ and R₂ may be the same or different and represent hydrogen, or a hydroxy protecting group,
by reacting a compound of general structure VII or VIII, 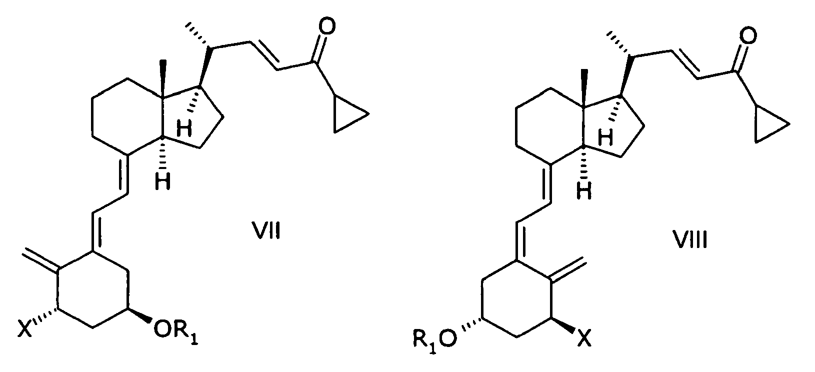 wherein R₁ and R₂ are as defined above,
with sulphur dioxide.

In a still further aspect, this invention relates to a method of reacting the mixture of compounds of general structure IVa and IVb,
wherein X represents either hydrogen or OR₂,
and wherein R₁ and R₂ may be the same or different and represent hydrogen, or a hydroxy protecting group,
which is enriched with IVa, in the presence of a base to give a mixture of compounds of general structure Va and Vb, which is enriched with Va,
wherein X, R₁, and R₂ are as defined above.

In a still further aspect, this invention relates to a method for producing calcipotriol {(5Z, 7E, 22E, 24S)-24-cyclopropyl-9,10-secochola-5,7,10(19),22-tetraene-1α-3β-24-triol} or calcipotriol monohydrate comprising any one of the methods above.

In a still further aspect, this invention relates to a compound of general structure IIIa or IIIb, or mixtures thereof, 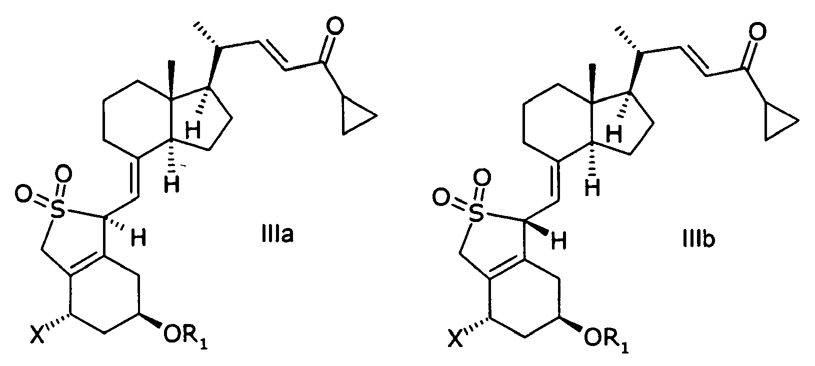 wherein X represents either hydrogen or OR₂,
and wherein R₁ and R₂ may be the same or different and represent hydrogen, or a hydroxy protecting group.

In a still further aspect, this invention relates to a compound of general structure IVaa, IVab, IVba, IVbb, IVb, or mixtures thereof, 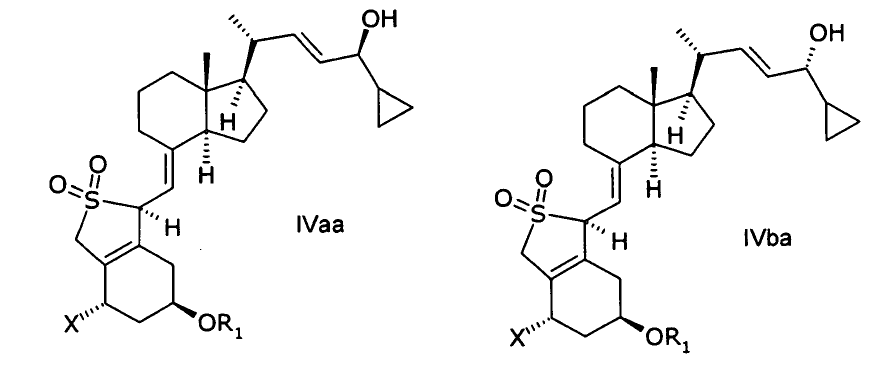 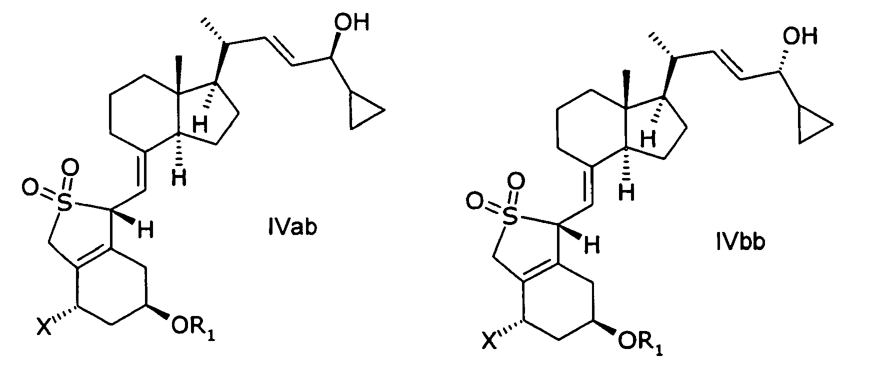 wherein X represents either hydrogen or OR₂,
and wherein R₁ and R₂ may be the same or different and represent hydrogen, or a hydroxy protecting group.

In a still further aspect, this invention relates to the use of a compound of general structure IIIa, IIb, IVaa, IVba, IVab, IVbb as an intermediate in the manufacture of calcipotriol or calcipotriol monohydrate.

### DETAILED DESCRIPTION OF THE INVENTION

### Definitions

As used herein a "hydroxy protecting group" is any group which forms a derivative that is stable to the projected reactions wherein said hydroxy protecting group can be selectively removed by reagents that do not attack the regenerated hydroxy group.
Said derivative can be obtained by selective reaction of a hydroxy protecting agent with a hydroxy group. Silyl derivatives, such as *tert*-butyldimethylsilyl forming silyl ethers are examples of hydroxy protecting groups. Silyl chlorides such as *tert*-butyldimethylsilyl chloride (TBSCI), trimethylsilylchloride, triethylsilylchloride, diphenylmethylsilylchloride, triisopropylsilylchloride, and *tert*-butyldiphenylsilylchloride are examples of hydroxy protecting agents. Hydrogen fluoride, such as aqueous HF in acetonitrile, or tetra n-butylammonium fluoride are examples of reagents which can remove silyl groups. Other hydroxy protecting groups include ethers, such as tetrahydropyranyl (THP) ether, including alkoxyalkyl ethers (acetals), such as methoxymethyl (MOM) ether, or esters, such as chloroacetate ester, trimethylacetate, acetate or benzoate ester. Non-limiting examples of hydroxy protecting groups and methods of protection and removal, all included in the scope of this application, can for example be found in "Protective Groups in Organic Synthesis", 3rd ed., T. W. Greene & P. G. M. Wuts eds., John Wiley 1999 and in "Protecting Groups", 1st ed., P.J. Kocienski, G. Thieme 2000.

As used herein, "alkyl" is intented to mean a linear or branched alkyl group, which may be cyclic or acyclic, having one to twenty carbon atoms, preferably one to seven carbon atoms. The methyl group, ethyl group, n-propyl group, isopropyl group, pentyl group, hexyl group, and the *tert*-butyldimethyl group are non-limiting examples of alkyl groups.

As used herein "reducing agent" is intended to mean any agent capable of reducing, including enantioselectively or diastereoselectively reducing, the C-24 keto group of a compound of general structure III to give a compound of general structure IV. In one embodiment, the chiral reducing agent may reduce the C-24 keto group of a compound of general structure III to yield a mixture of compounds of general structure IV, wherein said mixture is enriched for the desired epimer IVa (preferably yielding the 24-S isomer). In another embodiment, the reducing agent may reduce the C-24 keto group of a compound of general structure III in the presence of a chiral auxiliary to yield a mixture of compounds of general structure IV, wherein said mixture is enriched for the desired epimer IVa (preferably yielding the 24-S isomer) The reducing agent may be chiral or non-chiral. Examples of reducing agents include, but are not limited to borane reducing agents, metallic hydrides, such as lithium aluminium hydride, sodium borohydride, or AlH₃, optionally in the presence of lanthanide salts (e.g. LaCl₃, CeBr₃, CeCl₃), or NaBH₃(OAc), Zn(BH₄)₂, and Et₃SiH. Other reducing agents include, but are not limited to, hydrogen in the presence of a catalyst, such as platinum or ruthenium, sodium in ethanol, isopropyl alcohol and aluminium isopropoxide, and zinc powder in water or alcohol.

As used herein "borane reducing agent" includes borane or any borane derivative, such as borane complexes with amines or ethers. Non-limiting examples of borane reducing agents e.g. include *N,N*-diethylaniline-borane, borane-tetrahydrofuran, 9-borabicyclononane (9-BBN), or borane dimethylsulfide.

As used herein, "chiral auxiliary" means any chiral compound or optically active catalyst, e.g. a compound comprising asymmetrically substituted carbon atoms or axially chiral compounds, or mixtures of chiral compounds and/or optically active catalysts, which will improve the yield of a compound of general structure IVa with respect to its epimer (increase the molar ratio IVa:IVb) in the reduction of a compound of general formula III with said reducing agent. Said chiral auxiliaries will thus be any compound which is capable of increasing the stereoselectivity, in the reduction reaction of a compound of general structure III in comparison to the yield or stereoselectivity for IVa without the chiral auxiliary present or involved. Non-limiting examples of chiral auxiliaries include chiral 1,2-amino-alcohols, such as chiral *cis*-1-amino-2-indanol derivatives, such as (*1S,2R*)-(-)-*cis*-1-amino-2-indanol, or *cis*-1-amino-1,2,3,4-tetrahydronaphthalen-2-ol, such as (*1S,2R*)-*cis*-1-amino-1,2,3,4-tetrahydronaphthalen-2-ol. Other examples are binaphthyl derivatives, such as (*R*)-2,2'-bis(diphenylphosphino)-1,1'-binaphthyl-ruthenium acetate 2,2'-dihydroxy-1,1'binaphthyl derivatives. Further examples include but are not limited to (R)-(+)-α,α-diphenyl-2-pyrrolidinmethanol, (R)-(+)-2-amino-4-methyl-1,1-diphenyl-1-pentanol, (R)-(-)-2-amino-3-methyl-1,1-diphenyl-1-butanol, (R)-(+)-2-amino-1,1,3-triphenyl-1-propanol, and (1R,2S)-(-)-2-amino-1,2-diphenyl ethanol.

As used herein, "inert solvent" means any organic solvent compatible with said reducing agent under the reaction conditions employed, or mixtures of such solvents. The choice of such solvent will depend on the specific reducing agent used. Non-limiting examples of inert solvents include hydrocarbons, such as toluene, and ethers, such as *tert*-butyl methyl ether or tetrahydrofuran.

A mixture of compounds of general structure IVa and IVb, which is enriched with IVa, means a mixture, optionally comprising other compounds or solvents, were the molar ratio (diastereomer ratio) of IVa/IVb is one (50:50) or larger than one, thus that the mixture contains at least 50% of the compound of general structure IVa (containing 50% or less of the compound of general structure IVb).

A mixture of compounds of general structure Va and Vb, which is enriched with Va, means a mixture, optionally comprising other compounds or solvents, were the molar ratio (diastereomer ratio) of Va/Vb is one (50:50) or larger than one, thus that the mixture contains at least 50% of the compound of general structure Va (containing 50% or less of the compound of general structure Vb).

A mixture of compounds of general structure VIa and VIb, which is enriched with VIa, means a mixture, optionally comprising other compounds or solvents, were the molar ratio (diastereomer ratio) of VIa/VIb is one (50:50) or larger than one, thus that the mixture contains at least 50% of the compound of general structure VIa (containing 50% or less of the compound of general structure VIb).

As used herein, "separating a compound" includes the purification and/or isolation of a compound, e.g. to at least 90% purity, such as to at least 95% purity, such as 97% purity, 98% purity, or 99% purity. The term "separating a compound" also includes the meaning of enhancing the concentration of the compound in a mixture of such compounds, optionally comprising solvents, such that the mixture is further enriched with a desired or preferred compound or isomer, such as an epimer, after said separation.

### Embodiments

In a currently most preferred embodiment of the present invention X represents OR₂.

In a currently preferred embodiment of the present invention R₁ and/or R₂ represent alkylsilyl, such as *tert*-butyldimethylsilyl, most preferably R₁ and R₂ are the same.

In another embodiment of the present invention R₁ and R₂ represent hydrogen.

In a currently preferred embodiment of the present invention the reducing agent is a borane reducing agent, such as *N,N*-diethylaniline-borane, borane-tetrahydrofuran, or borane dimethylsulfide.

The reducing step is carried out with a chiral reducing agent or in the presence of a chiral auxiliary.

In a currently preferred embodiment of the present invention the chiral auxiliary is a chiral 1,2-amino-alcohol, such as a chiral *cis*-1-amino-2-indanol derivative, such as (*1S,2R*)-(-)-*cis*-1-amino-2-indanol.

In a currently preferred embodiment of the present invention the reducing step is carried out at a temperature between 10-20°C, in particular 15-20°C.

In another embodiment of the present invention the molar ratio (diastereomer ratio IVa/IVb) of a mixture of compounds of general structure IVa and IVb, which is enriched with IVa, is larger than 55:45, such as 56:44, such as 57:43, such as 59:41, such as 60:40, such as 63:37, such as 65:35, such as 68:32, such as 70:30, such as 72:28, such as 73:27, such as 74:26, such as 75:25, such as 76:24, such as 77:23, such as 78:22, such as 79:21, such as 80: 20.

In another embodiment of the present invention the molar ratio (diastereomer ratio Va/Vb) of a mixture of compounds of general structure Va and Vb, which is enriched with Va, is larger than 55:45, such as 56:44, such as 57:43, such as 59:41, such as 60:40, such as 63:37, such as 65:35, such as 68:32, such as 70:30, such as 72:28, such as 73:27, such as 74:26, such as 75:25, such as 76:24, such as 77:23, such as 78:22, such as 79:21, such as 80: 20.

In another embodiment of the present invention the molar ratio (diastereomer ratio VIa/VIb) of a mixture of compounds of general structure VIa and VIb, which is enriched with VIa, is larger than 55:45, such as 56:44, such as 57:43, such as 59:41, such as 60:40, such as 63:37, such as 65:35, such as 68:32, such as 70:30, such as 72:28, such as 73:27, such as 74:26, such as 75:25, such as 76:24, such as 77:23, such as 78:22, such as 79:21, such as 80: 20.

In one embodiment of the present invention, the compound of general structure Va is separated, e.g. by chromatography, from the mixture of compounds of general structure Va and Vb which is enriched with Va, wherein X, R₁ and R₂ are as defined above in (step (c)).

In another embodiment of the present invention, the compound of general structure VIa is separated, by chromatography, from the mixture of compounds of general structure VIa and VIb which is enriched with VIa, wherein X, R₁ and R₂ are as defined above in (step (g)).

### Synthetic Methods

The compounds of general structure III can for example be synthesized via Diels-Alder reaction by treatment of a compound of general structure VII or VIII with sulphur dioxide. The sulphur dioxide used can be liquid, gaseous or being dissolved in a suitable solvent. Suitable solvents for the Diels-Alder reaction are all solvents, which are compatible with the reaction conditions, such as alkanes, such as hexane or heptane, hydrocarbons, such as xylenes, toluene, ethers, such as diethyl ether or methyl-*tert-*butyl ether (MTBE), acetates, such as ethyl acetate or 2-propyl acetate, halogenated solvents such as dichloromethane, or mixtures of said solvents. In a preferred embodiment the solvent is toluene. In another preferred embodiment the solvent is a mixture of a water immiscible solvent and water, such as toluene and water. The reaction can also be carried out in neat sulphur dioxide without a solvent. A suitable reaction temperature of the process is -50°C to 60 °C, such as -30°C to 50°C, such as - 15°C to 40°C, such as -5°C to 30°C, such as 0°C to 35°C, such as 5°C to 30°C most such as 10°C to 25°C, such as 15°C to 20°C. In one embodiment the sulphur dioxide is used in excess (mol/mol), such as 5-100 molar excess, such as 7-30 molar excess, such as 10-15 molar excess. Any excess of unreacted sulphur dioxide may be removed from the reaction mixture by e.g. washing with aqueous base, such as aqueous sodium hydroxide or by distilling the sulphur dioxide off, optionally together with a solvent, optionally under reduced pressure. The compounds of general structure III are usually obtained as a mixture of their epimers IIIa and IIIb.

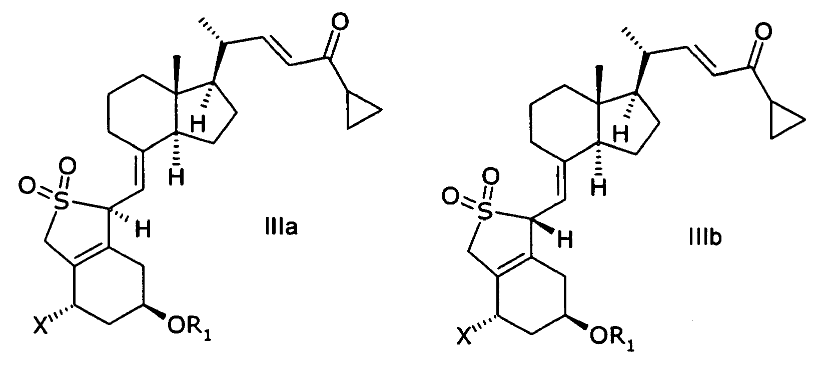

The molar ratio IIIa/IIIb of the mixture of the epimers obtained in the Diels-Alder reaction will depend on the groups X, R₁ and R₂ and the reaction conditions used. The present invention includes mixtures of all possible compositions (molar ratio IIIa/IIIb), such as 1:99, such as 2:98, such as 3:97, such as 4:96, such as 5:95, such as 10:90, such as 85:15, such as 80:20, such as 75:25, such as 30:70, such as 35:65, such as 40:60, such as 45:55, such as 50:50, such as 55:45, such as 60:40, such as 65:35, such as 70:30, such as 75:25, such as 80: 20, such as 85:10, such as 90:10, such as 95:5, such as 96:4, such as 97:3, such as 98:2, such as 99:1.

The general formula III includes mixtures of all possible compositions (molar ratio IIIa/IIIb) as above. In an embodiment of the present invention, compounds IIIa and IIIb are used as a mixture, as indicated in the general formula III in the following reduction step. The mixture of IIIa and IIIb may optionally be purified or separated, such as by chromatography or crystallisation. In another embodiment compound IIIa is used in the following reduction step. In yet another embodiment compound IIIb is used in the following reduction step.

Compounds of general structure VII can for example be synthesised according to methods disclosed for example by M. J. Calverley, Tetrahedron, Vol. 43, No. 20, pp. 4609-4619, 1987 or in WO 87/00834 and references cited therein. For example, compound VII, wherein X is OR₂ and both R₁ and R₂ are *tert*-butyldimethylsilyl which is described in these references can be deprotected with aqueous hydrofluoric acid in acetonitrile to give a mixture of compounds wherein X is OR₂ and either R₁ or R₂ are hydrogen, or to give a compound wherein X is OR₂ and R₁ and R₂ are both hydrogen. This mixture of compounds can for example be separated by chromatography or crystallised as generally described herein. By reaction of said compounds of general structure VII, wherein R₁ and/or R₂ are hydrogen with a suitable protecting agent, new groups R₁ and/or R₂ can be introduced. Depending on the stoichiometry of the protecting agent used and the reaction conditions, mixtures of unprotected, monoprotected, and diprotected compounds can be obtained. Any intermediate of a mixture wherein X is OR₂ and one of R₁ or R₂ is hydrogen can then be isolated by chromatography and reacted with suitable protecting agent different from the first one used, to give compounds of general structure VII, wherein X is OR₂ and R₁ is different from R₂.

Compounds of general structure VII wherein X is hydrogen and R₁ is hydrogen or a hydroxy protecting group can for example be prepared starting from compound 7a and/or 7b described by M. J. Calverley, Tetrahedron, Vol. 43, No. 20, p. 4610, 1987 and following analogues procedures and general synthetic methods as above and as described in the above cited references.

The reducing process of the present invention can for example be carried out by reacting a prochiral ketone of general structure III with a chiral borane reducing agent or a borane reducing agent in the presence of a chiral auxiliary. The process results in the enantioselective/diastereoselective reduction of the prochiral ketone, such that one of the two possible epimers IVa or IVb is formed in preference to the corresponding epimer. The degree of enantioselectivity/diastereoselectivity will depend on the reducing agent used, the chiral auxiliary and the reaction conditions.

The reduction reaction of a compound of general structure III is usually carried out in a temperature interval between -80°C to 70 °C, such as -40°C to 60°C, such as -15°C to 50°C, such as -5°C to 40°C, e.g. 0°C to 5°C or 5°C to 35°C. In one embodiment the remperature interval is between 10°C to 30°C, such as 15°C to 25°C, such as 15°C to 20°C. The optimum temperature will depend on the specific reaction condition and reagents used. In one embodiment of the present invention, the reaction mixture is immediately cooled to 0-10°C after completion to avoid the formation of by-products. If *N,N*-diethylaniline is used as reducing agent, the *N,N*-diethylaniline formed can be easily removed from the reaction mixture by extraction with aqueous hydrochloric acid. One molar equivalent with respect to the base to be extracted of 1M hydrochloric acid is preferred.

The reducing agent, optionally dissolved or mixed with an inert solvent, may be added to the compound of general structure III optionally dissolved or mixed with an inert solvent, e.g. under an inert atmosphere, such as nitrogen. Alternatively the compound of general structure III, optionally dissolved or mixed with an inert solvent, may be added to the reducing agent, optionally dissolved or mixed with an inert solvent (reversed order).

In one embodiment of the present invention, the reducing agent is used in an equimolar amount or in molar excess to a compound of general structure III. In a specific embodiment of the present invention, the molar ratio of reducing agent/ compound of general structure III is 1.0-5.0. In a presently preferred embodiment, the molar ratio of reducing agent/ compound of general structure III is 1.8-3.0, such as 2.3-2.9, such as 2.5-2.7.

The chiral auxiliary may react with the reducing agent prior to the reduction *in situ* to form a chiral reducing agent or the chiral auxiliary may for example serve as a chiral ligand in a complex with the reducing agent, i.e. for example to give a chiral reducing agent. The present invention includes the use of such chiral reducing agents or chiral ligand-reducing agent complexes, which were prepared and isolated separately before being used for the reduction of a compound of general structure III.

The term "reducing agent in the presence of a chiral auxiliary" thus includes any chiral reducing agent. For example, the chiral auxiliary may react with a borane reducing agent prior to the reduction *in situ* to form a chiral borane reducing agent or the chiral auxiliary may serve as a chiral ligand in a borane complex. Examples of such chiral borane reducing agents are chiral oxaborolidines or oxazaborolidines, such as chiral oxazaborolidine reagents derived from (1R,2S)-*cis*-1-amino-2-indanol, (1S,2R)-*cis*-1-amino-2-indanol, (S)-prolinol, (R)-prolinol or B-(3-pinanyl)-9-borabicyclo[3.3.2]nonane (alpine-borane), or e.g. 5,5-diphenyl-2-methyl-3,4-propano-1,3,2-oxazaborolidine, (S)-2-methyl-CBS-oxazaborolidine, (R)-2-methyl-CBS-oxazaborolidine. The present invention therefore includes the use of such chiral reducing agents, such as chiral borane reducing agents, or chiral ligand-reducing agent complexes, such as chiral ligand-borane complexes, which were prepared and isolated before being used for the reduction of a compound of general structure III.

Another example of a chiral ligand in a complex with the reducing agent is the complex of LiAlH₄ and 2,2'-dihydroxy-1,1'binaphthyl.

The molar ratio of reducing agent/chiral auxiliary is typically in the range of 0.1-20.0, such as 0.4-10.0, such as 0.3-5.0, such as 0.5-4.5, such as 1.0-4.0, such as 1.9-3.1, such as 2.1-2.9, such as 2.3-2.7, e.g. 10.8, 5.4, 2.6, 2.5, or 1.6.

The chiral auxiliary may be present in catalytic amounts, such as substoichiometric, or equimolar or in molar excess referring to a compound of general structure III or to the reducing agent. E.g. the ratio of chiral auxiliary/compound III may be 0.25-2.5, such as 0.5-2.0, such as 0.8-1.3, such as 0.9-1.2, such as 1.0-1.1.

The selection of a particular enantiomer of the chiral auxiliary will determine the stereoselective orientation of the hydroxy group of the compound of general structure IV with respect to C-24. Chiral auxiliaries which predominantly yield the S-configuration at C-24 are preferred.

Borane-catalysed reactions were for example reviewed by Deloux and Srebnik [Chem. Rev. 93, 763, 1993]. Examples of efficient catalysts based on chiral modified borane can for example be found in [A. Hirao, J. Chem. Soc. Chem. Commun. 315, 1981; E.J. Corey, J. Am. Chem. Soc. 109, 7925, 1987].

Examples of the synthesis and/or use of e.g. 1,2- and 1,3-amino alcohols in stereoselective reduction with borane can e.g. be found in [E. Didier et al.; Tetrahedron 47, 4941-4958, 1991; C.H. Senanayake et al., Tetrahedron Letters, 36(42), 7615-18, 1995, EP 0698028, EP 0640089, EP 0305180, WO 93/23408, WO 94/26751]. The synthesis and/or use of chiral *cis*-1-amino-2-indanol derivatives in borane reductions can e.g. be found in [C.H. Senanayake, Aldrichimica Acta, 31 (1), 1-15, 1998; A.K. Ghosh et. al., Synthesis, 937-961, 1998; Y. Hong et. al., Tetrahedron Letters, 35(36), 6631-34, 1994; B. Di Simone, Tetrahedron Asymmetry, 6(1) 301-06, 1995; Y. Hong et al., Tetrahedron Letters, 36(36), 6631-34, 1994; R. Hett et al., Org. Process Res. & Dev., 2, 96-99, 1998; or EP 0763005], and references cited therein.

The methods for producing calcipotriol as described herein may be modified with regard to the order of the reaction steps, by omitting one or more reaction steps, or by introducing additional purification or reaction steps at any stage of the reaction sequence. The present invention includes all such modifications.

The method for producing calcipotriol as described herein includes further all variants, where the hydroxy protecting groups R₁ and/or R₂ for compounds or intermediates, where R₁ and/or R₂ are not hydrogen, are removed at any stage of the reaction sequence. Compounds or intermediates, wherein R₁ and/or R₂ are hydrogen may be protected with protecting agents at any stage of the reaction sequence, including protecting agents which yield different protecting groups than those removed earlier in the reaction sequence.

Compounds and intermediates of the present invention may comprise asymmetrically substituted (chiral) carbon atoms and carbon-carbon double bonds which may give rise to the existence of isomeric forms, e.g. enantiomers, diastereomers and geometric isomers.

Epimers are known as diastereomers that have opposite configuration (R or S) at only one of multiple tetrahedral stereogenic centres in molecules having multiple stereogenic centres, such as the vitamin D analogues to which the present invention is directed.

Designation of, for example, C-24 as the epimeric centre of a pair of enantiomers therefore implies that the configuration at the other stereogenic centres of the pair are identical.

The present invention relates to all isomeric forms, such as epimers, either in pure form or as mixtures thereof.

The indication of a specific conformation or configuration either in the formulas or the names of compounds or intermediates of the present invention shall indicate that this specific conformation or configuration is a preferred embodiment of the invention. The indication of a specific conformation or configuration either in the formulas or the names of compounds or intermediates of the present invention shall include any other isomer than specifically indicated, either in pure form or as mixtures thereof, as another embodiment of the present invention.

The indication of an unspecific conformation or configuration either in the formulas or the names of compounds or intermediates of the present invention shall indicate that a mixture of these specific conformations or configurations is a preferred embodiment of the invention. For example, the compound of general formula III is a mixture of the epimers of general formula IIIa and IIIb.

The indication of an unspecific conformation or configuration either in the formulas or the names or numbering of compounds or intermediates of the present invention shall include any specific isomer although not specifically indicated in pure form, e.g. as another embodiment of the present invention.

For example, the compound of general formula IVa includes the following two epimers IVaa and IVab.

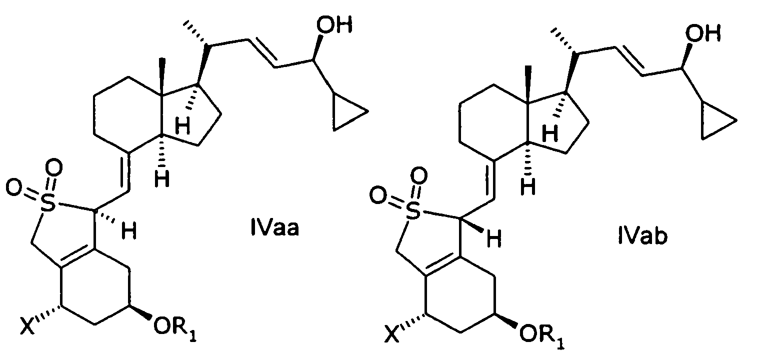

The meaning of compound of general formula III th us includes epimers IIIa and IIIb.

Pure stereoisomeric forms of the compounds and the intermediates of this invention may be obtained by the application of procedures known in the art, such as by chromatography or crystallisation, or by stereoselective synthesis.

The separation, isolation, and purification methods of the present invention include, but are not limited to chromatography, such as adsorption chromatography (including column chromatography and simulated moving bed (SMB)), crystallisation, or distillation. The separation, isolation, and purification methods may be used subsequently and in combination.

Column chromatography, useful for the separation of vitamin D analogues of the present invention is well known to those skilled in the art of pharmaceutical chemistry. The technique employs a column packed with a stationary phase, for example silica, such as pretreated silica onto which sample to be separated is loaded. The sample is then eluted with a suitable eluent. Elution can be isocratic or so-called solvent programmed (gradient), wherein the composition of the eluent is varied regularly (e.g. linearly) or irregularly (e.g. stepwise over time. Pretreated silica gel, well known to a person skilled in the art of chromatography, is a suitable stationary phase. Elution with 5% (v:v) ethyl acetate in hexane or heptane followed by neat ethyl acetate is but one example of an elution program that produces the desired separation. Other suitable eluents will be deduced by the skilled person through routine methods of development, e.g. by using mixtures of heptane and ethylacetate of suitable polarity.

For the chromatography step, any combination of stationary phase (packing) and eluent that is capable of resolving the mixture of C-24 epimers can be used. Such combinations can be readily determined by the skilled person by routine experimentation. An example of a preferred stationary phase is silica, such as treated silica.

The retro Diels-Alder reaction of the mixture of compounds of general structure IVa and IVb, which is enriched with IVa, in the presence of a base to give a mixture of compounds of general structure Va and Vb, which is enriched with Va, wherein X, R₁, and R₂ are as defined above, may be carried out in all solvents, which are compatible with the reaction conditions, such as alkanes, such as hexane or heptane, hydrocarbons, such as xylenes, toluene, ethers, such as diethyl ether or methyl-*tert*-butyl ether (MTBE), acetates, such as ethyl acetate or 2-propyl acetate, halogenated solvents such as dichloromethane, water or mixtures of said solvents.

Methods of said retro Diels Alder reaction are well known to a person skilled in the art of vitamin D synthesis (see e.g. M. J. Calverley, Tetrahedron, Vol. 43, No. 20, pp. 4609-4619, 1987 or in WO 87/00834).

Preferred solvents are toluene, *tert*-butyl methyl ether, water, or mixtures thereof. Suitable bases to be used in the retro Diels-Alder reaction include, but are not limited to NaHCO₃, KHCO₃, Na₂CO₃, or K₂CO₃. In one embodiment of the present invention, the base is aqueous NaHCO₃ and/or the retro Diels-Alder reaction is run above 60°C, such as above 70°C, such as between 70°C and 120 °C, such as between 74°C and 79 °C, such as between 72°C and 78 °C.

In one embodiment of the present invention, the temperature range of extractions and phase separations after the completion of the retro Diels-Alder reaction during reaction work-up are about 30°C-40°C.

Compounds of general structure VIII can be obtained by isomerisation of compounds of general structure VII.

Methods for the isomerisation of compounds of general formula Va and/or Vb to VIa and/or VIb, or VII to VIII, are well known to a person skilled in the art of vitamin D synthesis. Reaction conditions can e.g. be found in M. J. Calverley, Tetrahedron, Vol. 43, No. 20, pp. 4609-4619, 1987 or in WO 87/00834 and references cited therein. In a preferred embodiment of the present invention, the isomerisation is a photo isomerisation, e.g. with UV-light in the presence of a triplet sensitizer, e.g. anthracene or 9-acetylanthracene.

Compounds of general formula III, IV, V, VI, or VII, wherein X = hydrogen may be hydroxylated with a suitable hydroxylating agent, for example by a selenite mediated allylic hydroxylation, under the conditions developed by Hesse, e.g. with SeO₂ and N-methylmorpholine N-oxide in refluxing methanol and/or dichloromethane) [D.R. Andrews et al., J. Org. Chem., 1986, 51, 1637) or as described in M. J. Calverley, Tetrahedron, Vol. 43, No. 20, pp. 4609-4619, 1987 or in WO 87/00834, to give compounds of general formula III, IV, V, VI, or VII, wherein X = hydroxy (X=OR₂ and R₂ = hydrogen). The hydroxy groups of the starting materials may be protected with suitable protecting groups such as defined above by methods such as described above, for example to avoid undesired oxidation of said hydroxy groups.

Calcipotriol hydrate may be obtained by crystallisation of calcipotriol from aqueous solvents, such as for example by methods described in WO 94/15912.

### EXAMPLES

### General:

All chemicals, unless otherwise noted were from commercial sources. All melting points are uncorrected. For ¹H nuclear magnetic resonance (NMR) spectra (300 MHz) and ¹³C NMR (75.6 MHz) chemical shift values (δ) (in ppm) are quoted, unless otherwise specified; for deuteriochloroform solutions relative to internal tetramethylsilane (δ = 0.00) or chloroform (δ = 7.26) or deuteriochloroform (δ = 76.81 for ¹³C NMR) standard. The value of a multiplet, either defined (doublet (d), triplet (t), quartet (q)) or not (m) at the approximate mid point is given unless a range is quoted. All organic solvents used were of technical grade. Chromatography was performed on silica gel optionally using the flash technique. The TLC plates coated with silica gel were from Merck KGaA. Preferably the silica used for chromatography was from Merck KGaA Germany: LiChroprep® Si60 (15-25µm). Ethyl acetate, dichloromethane, hexane, n-hexane, heptane or appropriate mixtures of ethyl acetate, dichloromethane, methanol, and petroleum ether (40-60), hexane or heptane were used as eluents unless otherwise noted. All reactions may conveniently be carried out under an inert atmosphere, such as under a nitrogen atmosphere.

### Compounds of general structure III

### Examples 1:

### III: X=OR₂, R₁, R₂ = tert-butyldimethylsilyl

### 1(S),3(R)-bis(tert-butyldimethylsilyloxy)-20(R)-(3'-cyclopropyl-3'-oxoprop-1'(E)-enyl)-9,10-secopregna-5(E),7(E),10(19)-triene SO₂-adducts

20(R),1(S),3(R)-bis(*tert*-butyldimethylsilyloxy)-20-(3'-cyclopropyl-3'-oxoprop-1'(*E*)-enyl)-9,10-secopregna-5(*E*),7(*E*),10(19)-triene (prepared according to the method described by M. J. Calverley, Tetrahedron, Vol. 43, No. 20, pp. 4609-4619, 1987) (20.0g) was dissolved in toluene (210 ml) at 20°C followed by the addition of water (40 ml) and SO₂ (20 ml) with stirring. When the reaction was judged to be complete by HPLC {Column LiChrosorb Si 60 5 µm 250x4mm from Merck, 2 ml/min flow, detection at 270nm & mass detection, hexane/ethyl acetate 9:1 (v:v)}, usually after 2-2.5 hours, a mixture of sodium hydroxide (27.7%, 60 ml) and water (80 ml) was added at 10-18°C until pH 6 of the-reaction mixture. The toluene phase was separated and the solvent removed *in vacuo* without heating (preferably below 30°C) to give the two epimeric SO₂-adducts IIIa and IIIb as a solid mixture predominantly containing IIIa as checked by TLC. The two epimeric SO₂-adducts IIIa and IIIb could be separated by chromatography. Crystalline IIIa could be furthermore obtained by tituration of the solid mixture with methanol. ¹H NMR (CDCl₃) IIIa/ X=OR₂, R₁, R₂ = *tert*-butyldimethylsilyl = 6.73 (dd,1H), 6.14 (d,1H), 4.69 (d,1H), 4.62 (d,1H), 4.35 (s,1H), 4.17 (m,1H), 3.92 (d,1H), 3.58 (d,1H), 2.61 (m,1H), 2.29 (m,1H), 2.2 - 1.2 (m,16H), 1.11 (d,3H), 1.05 (m,2H), 0.90 (m,2H), 0.87 (s,9H), 0.85 (s,9H), 0.68 (s,3H), 0.06 (s,3H), 0.05 (s,3H), 0.04 (s,3H), 0.02 (s,3H) ppm.

### Preparation 1:

### VII: X=OR₂, R₁, R₂ = hydrogen

### 1(S),3(R)-dihydroxy-20(R)-(3'-cyclopropyl-3'-oxoprop-1'(E)-enyl)-9,10-secopregna-S(E),7(E),10(19)-triene

20(R),1(S),3(R)-bis(*tert*-butyldimethylsilyloxy)-20-(3'-cyclopropyl-3'-oxoprop-1'(*E*)-enyl)-9,10-secopregna-5(*E*),7(*E*),10(19)-triene which was obtained according to the method described by M. J. Calverley, Tetrahedron, Vol. 43, No. 20, p. 4614-4619, 1987 was dissolved in acetonitrile. Aqueous hydrofluoric acid (40%) was added and the mixture was stirred at room temperature for ca. 1 hour. The progress of the reaction could be conveniently checked by TLC using ethyl acetate as an eluent. Ethyl acetate was added to the reaction mixture and the mixture was washed with aqueous sodium hydrocarbonate solution. The organic phase was dried with over MgSO₄ and concentrated. The crystals (white needles) which formed were filtered off, washed with ethyl acetate, and dried in vacuo to give the title compound VII (X=OR₂, R₁, R₂ = hydrogen). ¹H NMR (CDCl₃) VII/ X=OR₂, R₁, R₂ = hydrogen = 6.77 (dd,1H), 6.57 (d,1H), 6.15 (d,1H), 5.88 (dd,1H), 5.13 (dd,1H), 4.98 (s,1H), 4.50 (m,1H), 4.23 (m,1H), 2.86 (m,2H), 2.29 (m,2H), 2.14 - 1.20 (m,16H), 1.14 (d,3H), 1.08 (m,2H), 0.89 (m,2H), 0.61 (s,3H) ppm.

### Example 2:

### III: X=OR₂, R₁, R₂ = hydrogen

### 1(S),3(R)-dihydroxy-20(R)-(3'-cyclopropyl-3'-oxoprop-1'(E)-enyl)-9,10-secopregna-5(E),7(E),10(19)-triene SO₂-adducts.

Same method as in Example 1, except that the starting material was 1(S),3(R)-dihydroxy-20(R)-(3'-cyclopropyl-3'-oxoprop-1'(*E*)-enyl)-9,10-secopregna-5(*E*),7(*E*),10(19)-triene from preparation 1. ¹H NMR (CDCl₃) III/ X=OR₂, R₁, R₂ = hydrogen δ = 6.80 (dd,1H), 6.15 (d,1H), 4.75 (m,2H), 4.5 - 3.9 (m,4H), 3.70 (d,1H), 2.60 (m,1H), 2.5 - 0.8 (m,25H), 0.68 (s,3H) ppm; ¹³C NMR (CDCl₃) III/ X=O₂, R1, R2 = hydrogen δ = 201.0, 152.1, 151.0, 133.7, 129.2, 128.3, 108.8, 67.3, 65.1, 63.6, 56.1, 55.9, 55.5, 46.5, 40.1, 39.9, 33.9, 29.8, 27.4, 23.9, 22.1, 19.5, 18.9, 12.2, 11.2 ppm.

### Preparation 2:

### VII: X=OR₂, R₁ = hydrogen, R₂ = tert-butyldimethylsilyl

### 1(S)- tert-butyldimethylsilyl-3(R)-hydroxy-20(R)-(3'-cyclopropyl-3'-oxoprop-1'(E)-enyl)-9,10-secopregna-5(E),7(E),10(19)-triene.

20(R),1(S),3(R)-bis(*tert*-butyldimethylsilyloxy)-20-(3'-cyclopropyl-3'-oxoprop-1'(*E*)-enyl)-9,10-secopregna-5(*E*),7(*E*),10(19)-triene was partially deprotected using the same deprotection conditions as used in Preparation 1 giving a mixture of unreacted startng material, two partially deprotected intermediates and the compound of Preparation 1. Purification by chromatography gave the pure title compound.

The ¹H NMR was found to be in accordance with the structure. ¹H NMR (CDCl₃) VII/X=OR₂, R₁ = hydrogen, R₂ = *tert*-butyldimethylsilyl δ = 6.75 (dd,1H), 6.50 (d,1H), 6.14 (d,1H), 5.84 (d,1H), 5.00 (s,1H), 4.92 (s,1H), 4.47 (t,1H), 4.22 (m,1H), 2.85 (dd,1H), 2.62 (dd,1H), 2.43 (dd,1H), 2.29 (m,1H), 2.15 - 1.15 (m,15H), 1.11 (d,3H), 1.06 (m,2H), 0.87 (s,9H), 0.86 (m,2H), 0.59 (s,3H), 0.06 (s,3H), 0.04 (s,3H) ppm.

### Example 3:

### III: X=OR₂, R₁ = hydrogen, R₂ = tert-butyldimethylsilyl

### 1(S)- tert-butyldimethylsilyl-3(R)-hydroxy-20(R)-(3'-cyclopropyl-3'-oxoprop-1'(E)-enyl)-9,10-secopregna-5(E),7(E),10(19)-triene SO₂-adducts.

Same method as in Example 1, except that the starting material was 1(S)- *tert*-butyldimethylsilyl-3(R)-hydroxy-20(R)-(3'-cyclopropyl-3'-oxoprop-1'(*E*)-enyl)-9,10-secopregna-5(*E*),7(*E*),10(19)-triene from preparation 2. ¹³C NMR (CDCl₃) III/X=OR₂, R₁ = hydrogen, R₂ = *tert*-butyldimethylsilyl δ = 200.3, 151.5, 150.4, 132.0, 129.5, 128.0, 108.5, 66.8, 65.5, 63.8, 56.1, 55.9, 55.2, 46.2, 39.8, 33.6, 29.5, 27.2, 25.4, 23.7, 21.8, 19.2, 18.5, 17.7, 11.8, 10.7, -4.7, -5.2 ppm; ¹H NMR (CDCl₃) IIIb/X=OR₂, R₁ = hydrogen, R₂ = *tert*-butyldimethylsilyl δ = 6.75 (dd,1H), 6.14 (d,1H), 4.80 (d,1H), 4.65 (d,1H), 4.43 (m,1H), 4.25 (m,1H), 3.92 (d,1H), 3.63 (dd,1H), 2.60 (d,1H), 2.5 - 1.2 (m,18H), 1.12 (d,3H), 1.06 (m,2H), 0.88 (s,9H), 0.87 (m,2H), 0.59 (s,3H), 0.09 (s,3H), 0.07 (s,3H) ppm.

### Preparation 3:

### VII: X=OR₂, R₁ = COCMe₃, R₂ = tert-butyldimethylsilyl

### 1(S)- tert-butyldimethylsilyl -3(R)-trimethylacetoxy-20(R)-(3'-cyclopropyl-3'-oxoprop-1'(E)-enyl)-9,10-secopregna-5(E),7(E),10(19)-triene

1(S)- *tert*-butyldimethylsilyl-3(R)-hydroxy-20(R)-(3'-cyclopropyl-3'-oxoprop-1'(*E*)-enyl)-9,10-secopregna-5(*E*),7(*E*),10(19)-triene from Preparation 2 may be reacted with trimethylacetic acide chloride in the presence of triethylamine in dichloromethane.

The obtained raw product may be purified by chromatography to give the pure title compound.

### Preparation 4:

### VII: X=OR₂, R₁ = COCMe₃, R₂ = hydrogen

### 1(S)-hydroxy-3(R)-trimethylacetoxy-20(R)-(3'-cyclopropyl-3'-oxoprop-1'(E)-enyl)-9,10-secopregna-5(E),7(E),10(19)-triene.

1(S)- *tert*-butyldimethylsilyl -3(R)-trimethylacetoxy-20(R)-(3'-cyclopropyl-3'-oxoprop-1'(*E*)-enyl)-9,10-secopregna-5(*E*),7(*E*),10(19)-triene may be deprotected using the same deprotection conditions as used in Preparation 1. The obtained raw product may be purified by chromatography to give the pure title compound.

### Example 4:

### III: X=OR₂, R₁ = COCMe₃, R₂ = hydrogen

### 1(S)-hydroxy-3(R)-trimethylacetoxy-20(R)-(3'-cyclopropyl-3'-oxoprop-1'(E)-enyl)-9,10-secopregna-5(E),7(E),10(19)-triene SO₂-adducts.

Same method as in example 1, except that the starting material was 1(S)-hydroxy-3(R)-trimethylacetoxy-20(R)-(3'-cyclopropyl-3'-oxoprop-1'(*E*)-enyl)-9,10-secopregna-5(*E*),7(*E*),10(19)-triene from preparation 4. ¹³C NMR (CDCl₃) IIIa/ X=OR₂, R₁ = COCMe₃, R₂ = hydrogen δ = 200.4, 177.6, 151.6, 150.9, 132.8, 129.3, 128.1, 108.8, 66.9, 66.3, 64.6, 55.8, 55.5, 55.3, 46.3, 39.9, 38.5, 36.3, 30.2, 29.6, 27.2, 26.9, 23.7, 21.8, 19.3, 18.6, 11.9, 10.8 ppm.

### Example 5:

### III: X=OR₂, R₁ = COCMe₃, R₂ = tert-butyldimethylsilyl

### 1(S)- tert-butyldimethylsilyl -3(R)-trimethylacetoxy-20(R)-(3'-cyctopropyl-3'-oxoprop-1'(E)-enyl)-9,10-secopregna-5(E),7(E),10(19)-triene SO₂-adducts.

Same method as in Example 1, except that the starting material was 1(S)- *tert*-butyldimethylsilyl -3(R)-trimethylacetoxy-20(R)-(3'-cyclopropyl-3'-oxoprop-1'(*E*)-enyl)-9,10-secopregna-5(*E*),7(*E*),10(19)-triene e.g. obtainable from Preparation 3.

### Preparation 5:

### VII: X=OR₂, R₁ = COMe, R₂ = hydrogen

### 1(S)-hydroxy-3(R)-acetoxy-20(R)-(3'-cyclopropyl-3'-oxoprop-1'(E)-enyl)-9,10-secopregna-5(E),7(E),10(19)-triene.

1(S),3(R)-dihydroxy-20(R)-(3'-cyclopropyl-3'-oxoprop-1'(*E*)-enyl)-9,10-secopregna-5(E),7(E),10(19)-triene (VII: X=OR₂, R₁, R₂ = hydrogen) from Preparation 1 may be reacted with one equivalent acetylchloride in the presence of triethylamine. The mixture of products may be purified by chromatography on silica to give the pure title compound.

### Example 6:

### III: X=OR₂, R₁ = COMe, R₂ = hydrogen

### 1(S)-hydroxy-3(R)-acetoxy-20(R)-(3'-cyclopropyl-3'-oxoprop-1'(E)-enyl)-9,10-secopregna-5(E),7(E),10(19)-triene SO₂-adducts.

Same method as in Example 1, except that the starting material was 1(S)-hydroxy-3(R)-acetoxy-20(R)-(3'-cyclopropyl-3'-oxoprop-1'(*E*)-enyl)-9,10-secopregna-5(*E*),7(*E*),10(19)-triene (VII: X=OR₂, R₁ = COMe, R₂ = hydrogen) from obtainable from Preparation 5. ¹³C NMR (CDCl₃) IIIa/ X=OR₂, R₁ = COMe, R₂ = hydrogen δ = 200.5, 170.3, 151.6, 150.9, 132.8, 129.2, 128.1, 108.3, 66.8, 66.4, 64.6, 55.9, 55.7, 55.3, 46.3, 39.9, 36.4, 30.4, 29.6, 27.2, 23.7, 21.8, 21.0, 19.3, 18.6, 11.9, 10.8.

### Example 7:

### III: X=OR₂, R₁ = COMe, R₂ = tert-butyldimethylsilyl

### 1(S)- tert-butyldimethylsilyl-3(R)-acetoxy-20(R)-(3'-cyclopropyl-3'-oxoprop-1'(E)-enyl)-9,10-secopregna-5(E),7(E),10(19)-triene SO₂-adducts.

Same method as in Example 1, except that the starting material was 1(S)- *tert*-butyldimethylsilyl-3(R)-acetoxy-20(R)-(3'-cyclopropyl-3'-oxoprop-1'(*E*)-enyl)-9,10-secopregna-5(*E*),7(*E*),10(19)-triene. ¹H NMR (CDCl₃) IIIa/ X=OR₂, R₁ = COMe, R₂ = *tert*-butyldimethylsilyl δ = 6.75 (dd,1H), 6.16 (d,1H), 5.20 (m,1H), 4.71 (s,2H), 4.33 (s,1H), 3.95 (d,1H), 3.60 (d,1H), 2.61 (m,1H), 2.31 (m,2H), 2.15 - 1.2 (m,15H), 2.03 (s,3H), 1.11 (d,3H), 1.07 (m,2H), 0.89 (m,2H), 0.88 (s,9H), 0.68 (s,3H), 0.08 (s,3H), 0.07 (s,3H) ppm.

### Example 8:

### III: X= hydrogen, R₁ = tert-butyldimethylsilyl

### 3(R)-tert-butyldimethylsilyloxy-20(R)-(3'-cyclopropyl-3'-oxoprop-1'(E)-enyl)-9,10-secopregna-5(E),7(E),10(19)-triene SO₂-adducts

The starting material VII, X=hydrogen, R₁ = *tert*-butyldimethylsilyl (prepared according to the methods described by M. J. Calverley, Tetrahedron, Vol. 43, No. 20, pp. 4609-4619, 1987) (38.5 g) was dissolved in toluene (550 ml) at 20°C followed by the addition of water (105 ml) and SO₂ (53 ml) with stirring. When the reaction was judged to be complete by HPLC {Column LiChrosorb Si 60 5 µm 250x4mm from Merck, 2 ml/min flow, detection at 270nm & mass detection, hexane/ethyl acetate 9:1 (v:v)}, usually after 2-2.5 hours, a mixture of sodium hydroxide (27.7%, 150 ml) and water (480 ml) was added at 10-18°C until pH 6 of the reaction mixture. The toluene phase was separated and the solvent removed *in vacuo* without heating (preferably below 30°C) to give two epimeric SO₂-adducts IIIa and IIIb (X= hydrogen, R₁ = *tert*-butyldimethylsilyl) as a solid mixture predominantly containing IIIa as checked by TLC. The two epimeric SO₂-adducts could be separated by chromatography. Crystalline IIIa could be furthermore obtained by tituration of the solid mixture with methanol. ¹³C NMR (CDCl₃) (III: X= hydrogen, R₁ = *tert*-butyldimethylsilyl, mixture of isomers IIIa and IIIb): 200.3, 151.6, 151.4, 149.8, 149.2, 130.5, 130.1, 128.3, 128.1, 126.6, 126.3, 110.5, 110.0, 67.4, 66.7, 66.6, 66.3, 58.0, 57.9, 55.8, 55.6, 55.3, 55.2, 46.3, 45.5, 39.9, 39.7, 34.4, 34.1, 33.9, 31.4, 30.8, 30.5, 29.6, 29.1, 27.3, 27.1, 26.7, 25.6, 25.1, 24.4, 24.1, 23.6, 23.2, 22.4, 21.9, 21.9, 19.4, 19.3, 18.6, 18.4, 17.9, 17.9, 13.9, 12.2, 11.9, 10.8, -5.0.

### Compounds of general structure IV

### Example 9:

### SO₂-adduct of 1(S),3(R)-bis(tert-butyl-dimethylsilyloxy)-20(R)-(3'-cyclopropyl-3'(S)-hydroxyprop-1'(E)-enyl)-9,10-secopregna-5(E),7(E),10(19)-triene

### (IVa: X=OR₂, R₁, R₂ = tert-butyldimethylsilyl), and

### SO₂-adduct of 1(S),3(R)-bis(tert-butyl-dimethylsilyloxy)-20(R)-(3'-cyclopropyl-3(R)'-hydroxyprop-1'(E)-enyl)-9,10-secopregna-5(E),7(E),10(19)-triene

### (IVb: X=OR₂, R₁, R₂ = tert-butyldimethylsilyl)

(*1S,2R*)-(-)-*cis*-1-amino-2-indanol (5.0g) was mixed with MTBE (160 ml) under a nitrogen atmosphere at 15-25°C followed by the addition of *N,N*-diethylaniline-borane (16.0 ml) at that temperature. The mixture was stirred until no more evolution of hydrogen could be observed. The mixture of SO₂-adducts of 1(S),3(R)-bis(*tert*-butyldimethylsilyloxy)-20(R)-(3'-cyclopropyl-3'-oxoprop-1'(*E*)-enyl)-9,10-secopregna-5(E),7(E),10(19)-triene (III: X=OR₂, R₁, R₂ = *tert*-butyldimethylsilyl) obtained in Example 1 was dissolved in toluene (160 ml) and MTBE (80 ml). This solution was added dropwise to the borane containing mixture at 15-25°C. The mixture was stirred for ca. 30-60 minutes after complete addition and then quenched with saturated aqueous NaHCO₃ (110 ml) at 10-15°C. The organic phase was separated and washed with 1 M hydrochloric acid (100 ml) at 0-10°C followed by washing with saturated aqueous NaHCO₃ (100 ml). The organic phase contained the SO₂-adducts of 1(S),3(R)-bis(*tert-*butyl-dimethylsilyloxy)-20(R)-(3'-cyclopropyl-3(S)'-hydroxyprop-1'(*E*)-enyl)-9,10-secopregna-5(*E*),7(*E*),10(19)-triene (IVa: X=OR₂, R1, R2 = *tert*-butyldimethylsilyl), and the SO₂-adducts of 1(S),3(R)-bis(tert-butyl-dimethylsilyloxy)-20(R)-(3'-cyclopropyl-3(R)'-hydroxyprop-1'(*E*)-enyl)-9,10-secopregna-5(*E*),7(*E*),10(19)-triene (IVb: X=OR₂, R₁, R₂ = *tert*-butyldimethylsilyl) in a molar ratio of 72-78 : 22-28 (IVa:IVb) as checked by HPLC-analysis of an aliquot after retro-Diels Alder reaction and analysis according to the method described in example 10}. Compound IVaa was isolated by chromatography on silica. ¹³C NMR (CDCl₃) IVa/ X=OR₂, R₁, R₂ = *tert*-butyldimethylsilyl δ = 150.6, 137.6, 132.3, 129.3, 128.8, 109.0, 76.9, 67.3, 65.8, 64.5, 56.2, 56.1, 55.9, 46.0, 40.5, 40.0, 39.6, 34.1, 29.6, 27.4, 25.6, 25.5, 23.8, 21.8, 20.3, 17.8, 17.7, 17.4, 11.8, 2.8, 1.7, -4.7, -5.0, -5.0, -5.2 ppm.

### Example 10:

### SO₂-adducts of 3(R)-tert-butyl-dimethylsilyloxy-20(R)-(3'-cyclopropyl-3'(S)-hydroxyprop-1'(E)-enyl)-9,10-secopregna-5(E),7(E),10(19)-triene

### (IVa: X=hydrogen, R₁ = tert-butyldimethylsilyl), and

### SO₂-adducts of 3(R)-tert-butyl-dimethylsilyloxy-20(R)-(3'-cyclopropyl-3'(R)-hydroxyprop-1'(E)-enyl)-9,10-secopregna-5(E),7(E),10(19)-triene

### (IVb: X=hydrogen, R₁ = tert-butyldimethylsilyl),

(*1S,2R*)-(-)-*cis*-1-amino-2-indanol (1.22 g, 1.08 eq.) was mixed with MTBE (36 ml) under a nitrogen atmosphere at 15-25°C followed by the addition of *N,N*-diethylaniline-borane (3.6 ml, 2.7 eq.) at that temperature. The mixture was stirred until no more evolution of hydrogen could be observed. The mixture of SO₂-adducts of 3(R)-*tert*-butyldimethylsilyloxy-20(R)-(3'-cyclopropyl-3'-oxoprop-1'(*E*)-enyl)-9,10-secopregna-5(*E*),7(*E*),10(19)-triene (III: X=hydrogen, R₁ = *tert*-butyldimethylsilyl) obtained in Example 8 (4.32 g) was dissolved in a mixture of MTBE (18 ml) and toluene (36 ml) at room temperature and then added dropwise to the borane containing mixture at 15-25°C over 15 min. The mixture was stirred for ca. 60 minutes after complete addition and then quenched with saturated aqueous NaHCO₃ (25 ml). The organic phase was separated and washed with 1 M hydrochloric acid (25 ml) at 0-10°C followed by washing with saturated aqueous NaHCO₃ (25 ml) at 10-20°C. The organic phase contained the SO₂-adducts of 3(R)-*tert*-butyl-dimethylsilyloxy-20(R)-(3'-cyclopropyl-3(S)'-hydroxyprop-1'(*E*)-enyl)-9,10-secopregna-5(*E*),7(*E*),10(19)-triene (IVa: X=hydrogen, R₁, = *tert*-butyldimethylsilyl), and the SO₂-adducts of 3(R)-tert-butyl-dimethylsilyloxy-20(R)-(3'-cyclopropyl-3(R)'-hydroxyprop-1'(*E*)-enyl)-9,10-secopregna-5(*E*),7(*E*),10(19)-triene (IVb: X=hydrogen, R₁ = *tert*-butyldimethylsilyl).

### Compounds of general structure V

### Example 11:

### 1(S),3(R)-bis(tert-butyl-dimethylsilyloxy)-20(R)-(3'-cyclopropyl-3(S)'-hydroxyprop-1'(E)-enyl)-9,10-secopregna-5(E),7(E),10(19)-triene

### (Va: X=OR₂, R₁, R₂ = tert-butyldimethylsilyl), and

### 1(S),3(R)-bis(tert-butyl-dimethylsilyloxy)-20(R)-(3'-cyclopropyl-3(R)'-hydroxyprop-1'(E)-enyl)-9,10-secopregna-5(E),7(E),10(19)-triene

### (Vb: X=OR₂, R₁, R₂ = tert-butyldimethylsilyl)

The organic phase from Example 9 containing the SO₂-adducts of IVa (X=OR₂, R₁, R₂ = *tert*-butyldimethylsilyl), and IVb (X=OR₂, R₁, R₂ = *tert*-butyldimethylsilyl) was stirred vigorously with saturated aqueous NaHCO₃ (110 ml) and then heated (bath temperature ca. 90 °C) where the MTBE was distilled off. Conveniently the retro Diels-Alder reaction could be checked by HPLC HPLC {Column LiChrosorb Si 60 250x4mm from Merck, 1 ml/min flow, detection at 270nm, hexane/ethyl acetate 9:1.5 (v:v)}. After completion (usually 2-2.5 hours), the reaction mixture was cooled to 30-40°C and the organic phase was separated, washed with saturated aqueous NaHCO₃ (110 ml) and water (100 ml). The solvent was removed *in vacuo* and the obtained oil (29 g) was dissolved in hexane (200 ml). The organic mixture was cooled to ca. -15°C, filtered over a short path of silica, and the remainder washed with hexane (ca. 100 ml). The hexane phase was washed with a mixture of methanol and water (1:2) and the organic solvent was removed *in vacuo*. The remaining oil, containing a mixture of 1(S),3(R)-bis(*tert*-butyldimethylsilyloxy)-20(R)-(3'-cyclopropyl-3(S)'-hydroxyprop-1'(*E*)-enyl)-9,10-secopregna-5(E),7(E),10(19)-triene (Va: X=OR₂, R₁, R₂ = *tert*-butyldimethylsilyl), and 1(S),3(R)-bis(*tert*-butyl-dimethylsilyloxy)-20(R)-(3'-cyclopropyl-3(R)'-hydroxyprop-1'(*E*)-enyl)-9,10-secopregna-5(*E*),7(*E*),10(19)-triene (Vb: X=OR₂, R₁, R₂ = *tert*-butyldimethylsilyl) in a molar ratio of a range of 72-78 : 22-28 (Va:Vb) as checked by HPLC {Column LiChrosorb Si 60 5 µm 250x4mm from Merck, 1 ml/min flow, detection at 270nm, n-heptane/2-propanol 100:0.25 (v:v): RT Va ca. 14.3 min, Vb: 11.9 min; or hexane/ethyl acetate 90:15 (v:v): RT Va ca. 7.6 min, Vb: 6.4 min}, was purified by chromatography as described earlier by M. J. Calverley, Tetrahedron, Vol. 43, No. 20, pp. 4609-4619, 1987 or in WO 87/00834, to give 10.9 g (98.9 % HPLC purity) of Va/ X=OR₂, R₁, R₂ = *tert*-butyldimethylsilyl after crystallisation from a mixture of hexane and methanol and a small amount of triethylamine (by slowly evaporating the hexane followed by cooling to - 15°C), in full accordance with the data described by M. J. Calverley in Tetrahedron, Vol. 43, No. 20, p. 4617, 1987 for compound 22. ¹³C NMR (CDCl₃) Va/ X=OR₂, R₁, R₂ = *tert-*butyldimethylsilyl δ = 153.4, 142.9, 137.9, 135.2, 128.7, 121.5, 116.3, 106.4, 77.1, 70.0, 67.0, 56.2, 55.8, 45.7, 43.7, 40.2, 39.8, 36.3, 28.7, 27.5, 25.6, 25.6, 23.3, 22.0, 20.3, 18.0, 17.9, 17.4, 12.1, 2.9, 1.6, -5.0, -5.0, -5.1; Vb/ X=OR₂, R₁, R₂ = *tert-*butyldimethylsilyl, δ = 153.5, 142.9, 137.6, 135.3, 128.7, 121.5, 116.3, 106.4, 76.8, 70.0, 67.0, 56.2, 56.0, 45.7, 43.8, 40.2, 39.7, 36.4, 28.7, 27.6, 25.7, 25.6, 23.3, 22.0, 20.3, 18.0, 17.9, 17.3, 12.1, 2.8, 1.6, -5.0, -5.1, -5.1 ppm.

### Example 12:

### 3(R)-tert-butyl-dimethylsilyloxy-20(R)-(3'-cyclopropyl-3(S)'-hydroxyprop-1'(E)-enyl)-9,10-secopregna-5(E),7(E),10(19)-triene

### (Va: X=hydrogen, R₁ = tert-butyldimethylsilyl), and

### 3(R)-tert-butyl-dimethylsilyloxy-20(R)-(3'-cyclopropyl-3(R)'-hydroxyprop-1'(E)-enyl)-9,10-secopregna-5(E),7(E),10(19)-triene

### (Vb: X=hydrogen, R₁ = tert-butyldimethylsilyl)

The organic solution from Example 10 containing the SO₂-adducts of IVa (X=hydrogen, R₁ = *tert*-butyldimethylsilyl), and IVb (X=hydrogen, R₁ = *tert*-butyldimethylsilyl) was stirred vigorously with saturated aqueous NaHCO₃ (25 ml) and then heated (bath temperature ca. 90 °C) where the MTBE was distilled off. Conveniently the retro Diels-Alder reaction could be checked by HPLC HPLC {Column LiChrosorb Si 60 250x4mm from Merck, 1 ml/min flow, detection at 270nm, hexane/ethyl acetate 9:1.5 (v:v)}. After completion (approx. 2 hours), the reaction mixture was cooled to 15-25°C and the organic phase was separated, washed with water (25 ml)., containing a mixture of Va:Vb (X=hydrogen, R₁ = *tert*-butyldimethylsilyl) in a molar ratio of (75:25) as checked by HPLC {Column LiChrosorb Si 60 5 µm 250x4mm from Merck, 1 ml/min flow, detection at 270nm, hexane/ethylacetate 90:15 (v:v): RT Vb: ca. 6.1 min, RT Va: ca. 7.4 min}. ¹H NMR (CDCl₃) Va/ X=hydrogen, R₁ = *tert*-butyldimethylsilyl δ = 6.45 (d,1H), 5.84 (d,1H), 5.46 (m,2H), 4.92 (s,1H), 4.63 (s,1H), 3.84 (m,1H), 3.42 (m,1H), 2.85 (d,1H), 2.64 (d,1H), 2.45 (m,1H), 2.32 - 1.18 (m,17H), 1.04 (d,3H), 0.98 (m,1H), 0.87 (s,9H), 0.56 (s,3H), 0.51 (m,2H), 0.32 (m,1H), 0.22 (m,1H), 0.05 (s,3H), 0.04 (s,3H); Vb/ X=hydrogen, R₁ = *tert*-butyldimethylsilyl δ = 6.45 (d,1H), 5.83 (d,1H), 5.47 (m,2H), 4.90 (s,1H), 4.62 (s,1H), 3.83 (m,1H), 3.45 (m,1H), 2.83 (d,1H), 2.62 (d,1H), 2.44 (m,1H), 2.24 (m,1H), 2.18 - 1.17 (m,16H), 1.03 (d,3H), 0.96 (m,1H), 0.86 (s,9H), 0.55 (s,3H), 0.50 (m,2H), 0.30 (m,1H), 0.20 (m,1H), 0.05 (s,3H), 0.04 (s,3H).

### Compounds of general structure VI

### Example 13:

### 1(S),3(R)-bis(tert-butyl-dimethylsilyloxy)-20(R)-(3'-cyclopropyl-3(S)'-hydroxyprop-1'(E)-enyl)-9,10-secopregna-5(Z),7(E),10(19)-triene

### (X=OR₂, VIa: R₁, R₂ = tert-butyldimethylsilyl)

1(S),3(R)-bis(*tert*-butyl-dimethylsilyloxy)-20(R)-(3'-cyclopropyl-3(S)'-hydroxyprop-1'(*E*)-enyl)-9,10-secopregna-5(*E*),7(*E*),10(19)-triene (Va: X=OR₂, R₁, R₂ = *tert-*butyldimethylsilyl) obtained in Example 11 was photosisomerised in toluene using a high pressure ultraviolet lamp at 20°C as described earlier by M. J. Calverley, Tetrahedron, Vol. 43, No. 20, pp. 4609-4619, 1987 or in WO 87/00834, except that 9-acetylanthracene was used instead of anthracene, to give 1(S),3(R)-bis(*tert*-butyldimethylsilyloxy)-20(R)-(3'-cyclopropyl-3(S)'-hydroxyprop-1'(*E*)-enyl)-9,10-secopregna-5(Z),7(E),10(19)-triene (VIa: X=OR₂, R₁, R₂ = *tert*-butyldimethylsilyl) after chromatography in full accordance with the data described by M. J. Calverley in Tetrahedron, Vol. 43, No. 20, p. 4618, 1987 for compound 28.

### Calcipotriol

### Example 14:

### 1(S),3(R)-dihydroxy-20(R)-(3'-cyclopropyl-3(S)'-hydroxyprop-1'(E)-enyl)-9,10-secopregna-5(Z),7(E),10(19)-triene

1(S),3(R)-bis(*tert*-butyl-dimethylsilyloxy)-20(R)-(3'-cyclopropyl-3(S)'-hydroxyprop-1'(*E*)-enyl)-9,10-secopregna-5(Z),7(*E*),10(19)-triene (VIa: X=OR₂, R₁, R₂ = *tert-*butyldimethylsilyl) obtained in Example 13 was deprotected using tetrabutyl ammonium fluoride in tetrahydrofuran at 60°C followed by chromatography, as described earlier by M. J. Calverley, Tetrahedron, Vol. 43, No. 20, pp. 4609-4619, 1987 or in WO 87/00834. Crystallisation from ethylacetate/hexane containing a few drops of triethylamine gave calcipotriol in full accordance with the data described by M. J. Calverley in Tetrahedron, Vol. 43, No. 20, p. 4618, 1987 for compound 4.

### Calcipotriol monohydrate

### Example 15:

The calcipotriol obtained in Example 14 was crystallised from ethyl acetate / water as described in WO 94/15912 to give calcipotriol monohydrate in full accordance with the characteristic data described in that patent.

### Diastereoselective reduction under various reducing conditions

### Example 16:

**Tabel 1:**

| | | | | |
|---|---|---|---|---|
| Diastereoselective reduction of compounds of general structure III, where X=OR₂ and R₁ and R₂ = *tert*-butyldimethylsilyl (mixture of 1(S),3(R)-bis(*tert*-butyldimethylsilyloxy)-20(R)-(3'-cyclopropyl-3'-oxoprop-1'(*E*)-enyl)-9,10-secopregna-5(*E*),7(*E*),10(19)-triene SO₂-adducts from Example 1 following a procedure analogous to Example 9 followed by cheletropic extrusion of sulphur dioxide following a procedure analogous to Example 11 to yield compounds of general structure. Va: X=OR₂, R₁, R₂ = *tert*-butyldimethylsilyl and Vb: X=OR₂, R₁, R₂ = *tert*-butyldimethylsilyl under various conditions (eq.= molar equivalents relative to III; MTBE = *tert*-butylmethyl ether; DEANB = *N,N*-diethylaniline borane). | | | | |

| **Chiral Auxiliary (eq.)** | **Reducing reagent (eq.)** | **Temp. (°C)** | **Solvent** | **Ratio Va:V b (%)** |
|---|---|---|---|---|
| (*1S,2R*)-(-)-*cis*-1-amino-2-indanol | DEANB | 15-20 | MTBE/ toluene | 72:28 |
| | (2.7eq.) | | | |
| (1.1 eq.) | | | | |
| (*1S,2R*)-(-)-*cis*-1-amino-2-indanol | DEANB | 20-25 | MTBE/ toluene | 72:28 |
| | (2.7eq.) | | | |
| (1.1 eq.) | | | | |
| (*1S,2R*)-(-)-*cis*-1-amino-2-indanol | DEANB | 10-15 | MTBE/ toluene | 70:30 |
| | (2.7eq.) | | | |
| (1.1 eq.) | | | | |
| (*1S,2R*)-(-)-*cis*-1-amino-2-indanol | DEANB (2.7eq.) | 15-20 | MTBE/ toluene | 72:28 |
| | | | | |
| (0.5 eq.) | | | | |
| (*1S,2R*)-(-)-*cis*-1-amino-2-indanol | DEANB | 15-20 | MTBE/ toluene | 56:44 |
| | (2.7eq.) | | | |
| (0.25 eq.) | | | | |
| (*1S,2R*)-(-)-*cis*-1-amino-2-indanol | DEANB | 15-20 | MTBE/ toluene | 59:41 |
| | (1.8 eq.) | | | |
| (1.1 eq.) | | | | |
| (*1S,2R*)-(-)-*cis*-1-amino-2-indanol | BH₃.THF | 15-20 | MTBE/ toluene | 75:25 |
| | (2.7 eq.) | | | |
| (1.1 eq.) | | | | |
| (*1S,2R*)-(-)-*cis*-1-amino-2-indanol | BH₃.SMe₂ | 15-20 | MTBE/ toluene | 73:27 |
| | (2.7 eq.) | | | |
| (1.1 eq.) | | | | |
| (*1S,2R*)-(-)-*cis*-1-amino-2-indanol | DEANB | 15-20 | THF | 63:37 |
| | (2.7eq.) | | | |
| (1.1 eq.) | | | | |
| (R)-(+)-α,α-Diphenyl-2-pyrrolidinmethanol | DEANB | 15-20 | MTBE/ toluene | 68:32 |
| | (2.7eq.) | | | |
| (1 eq.) | | | | |
| (R)-(+)-2-Amino-4-methyl-1,1-diphenyl-1-pentanol | DEANB | 15-20 | MTBE/ toluene | 72:28 |
| | (2.7eq.) | | | |
| (0.5 eq.) | | | | |
| (R)-(-)-2-Amino-3-methyl-1,1-diphenyl-1-butanol | DEANB | 15-20 | MTBE/ toluene | 76:24 |
| | (2.7eq.) | | | |
| (0.5 eq.) | | | | |
| (R)-(+)-2-amino-1,1,3-triphenyl-1-propanol | DEANB | 15-20 | MTBE/ | 74:26 |
| | (2.7eq.) | | toluene | |
| (0.5 eq.) | | | | |
| (1R,2S)-(-)-2-Amino-1,2-diphenyl ethanol | DEANB | 15-20 | MTBE/ | 57:43 |
| | (2.7eq.) | | toluene | |
| (0.5 eq.) | | | | |

## Claims

1. A method of reducing a compound of general structure III, 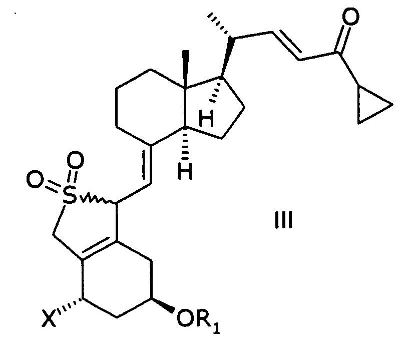 wherein X represents either hydrogen or OR₂,
and wherein R₁ and R₂ may be the same or different and represent hydrogen, or a hydroxy protecting group,
in an inert solvent with a chiral reducing agent or with a reducing agent in the presence of a chiral auxiliary,
to give a mixture of compounds of general structure IVa and IVb, 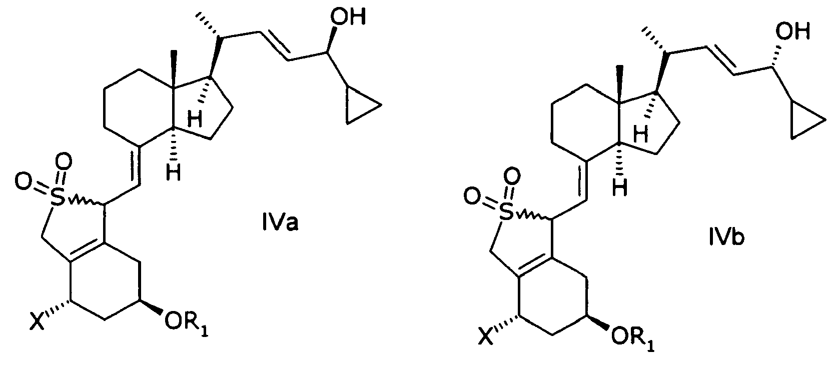 which is enriched with IVa, wherein X, R₁, and R₂ are as defined above.

2. A method for producing calcipotriol {(5Z, 7E, 22E, 24S)-24-cyclopropyl-9,10-secochola-5,7,10(19),22-tetraene-1α-3β-24-triol} or calcipotriol monohydrate comprising the steps of:
(a) reducing a compound of general structure III, 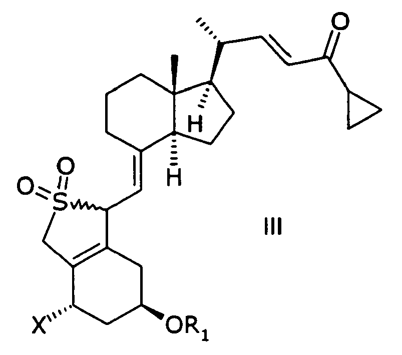 wherein X represents OR₂,
and wherein R₁ and R₂ may be the same or different and represent hydrogen or a hydroxy protecting group,
in an inert solvent with a chiral reducing agent or with a reducing agent in the presence of a chiral auxiliary,
to give a mixture of compounds of general structure IVa and IVb,
which is enriched with IVa, 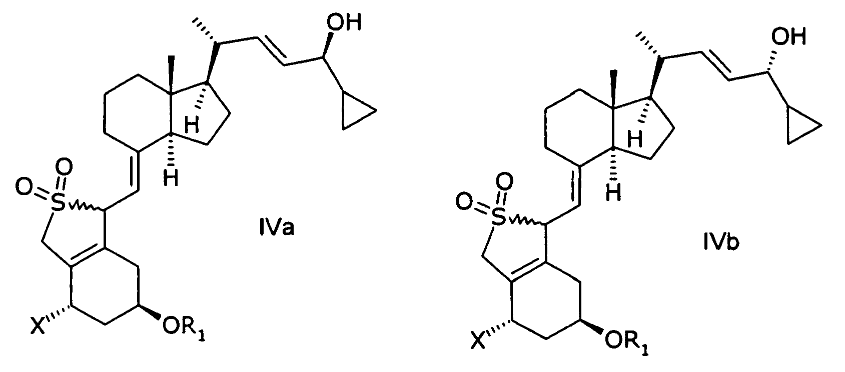 wherein X, R₁ and R₂ are as defined above;
(b) reacting the mixture of compounds of general structure IVa and IVb, which is enriched with IVa, in the presence of a base to give a mixture of compounds of general structure Va and Vb, which is enriched with Va, 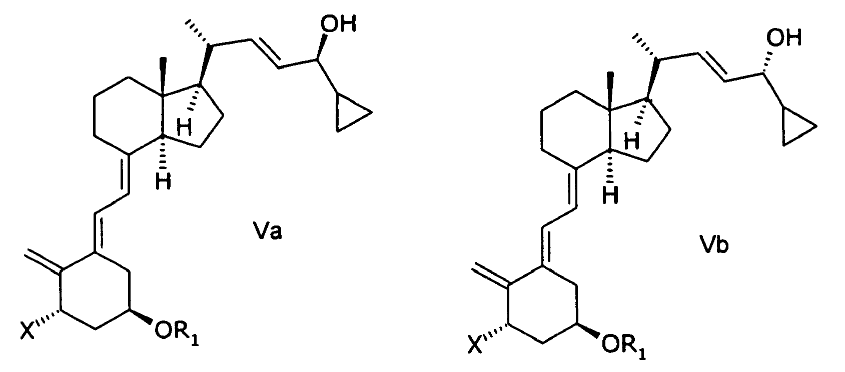 wherein X, R₁ and R₂ are as defined above;
(c) separating the compound of general structure Va from the mixture of compounds of general structure Va and Vb which is enriched with Va, wherein X, R₁ and R₂ are as defined above;
(d) isomerising the compound of general structure Va to the compound of general structure VIa, 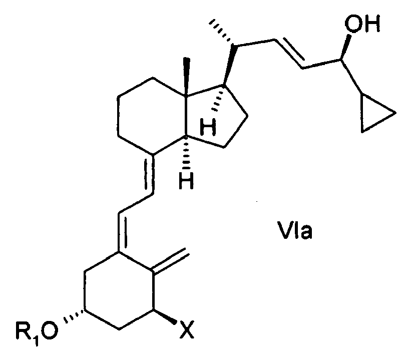 wherein X, R₁ and R₂ are as defined above; and
(e) when R₁ and/or R₂ are not hydrogen, removing the hydroxy protecting group(s) R₁ and/or R₂ of the compound of general structure VIa to generate calcipotriol or calcipotriol monohydrate.

3. A method for producing calcipotriol or calcipotriol monohydrate comprising steps (a) - (b) of claim 2 and further comprising the steps of:
(f) isomerising the mixture of compounds of general structure Va and Vb, wherein X, R₁ and R₂ are as defined in claim 2, which is enriched with Va, to a mixture of compounds of general structure VIa and VIb, which is enriched with VIa, 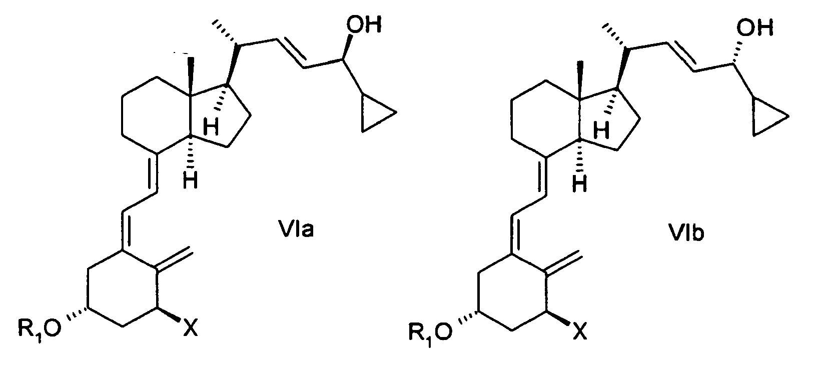 wherein X, R₁ and R₂ are as defined above;
(g) separating the compound of general structure VIa from the mixture of compounds of general structure VIa and VIb which is enriched with VIa, wherein X, R₁ and R₂ are as defined above;
(h) when R₁ and/or R₂ are not hydrogen, removing the hydroxy protecting group(s) R₁ and/or R₂ of the compound of general structure VIa to generate calcipotriol or calcipotriol monohydrate.

4. A method for producing calcipotriol {(5Z, 7E, 22E, 24S)-24-cyclopropyl-9,10-secochola-5,7,10(19),22-tetraene-1α-3β-24-triol} or calcipotriol monohydrate comprising the steps of:
(j) reducing a compound of general structure III, 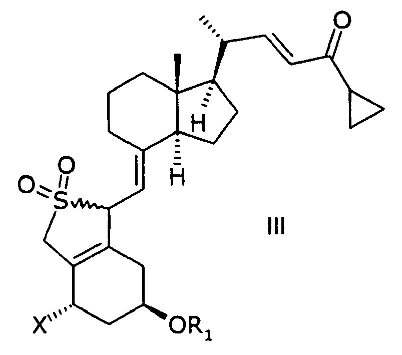 wherein X represents hydrogen,
and wherein R₁ represents hydrogen or a hydroxy protecting group,
in an inert solvent with a chiral reducing agent or with a reducing agent in the presence of a chiral auxiliary,
to give a mixture of compounds of general structure IVa and IVb,
which is enriched with IVa, 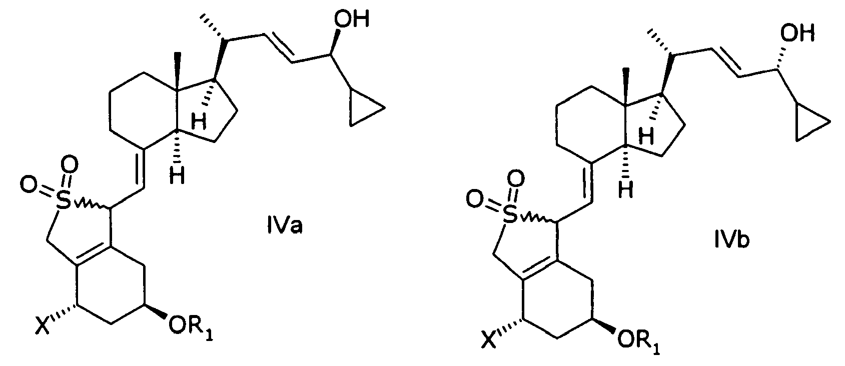 wherein X and R₁ are as defined above;
(k) reacting the mixture of compounds of general structure IVa and IVb, which is enriched with IVa, in the presence of a base to give a mixture of compounds of general structure Va and Vb, which is enriched with Va, 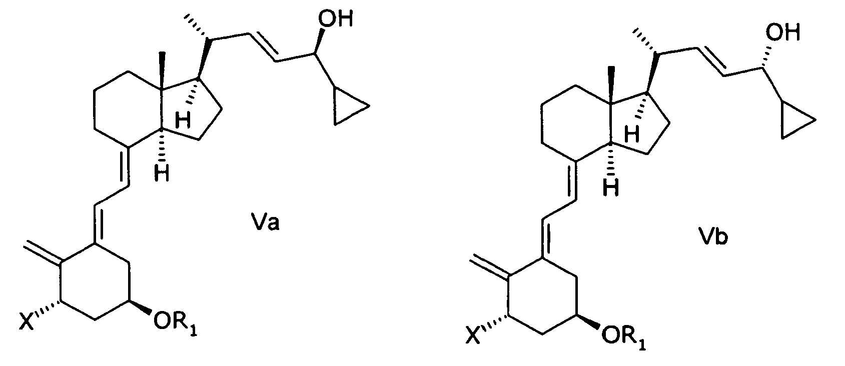 wherein X and R₁ are as defined above;
(I) separating the compound of general structure Va from the mixture of compounds of general structure Va and Vb which is enriched with Va, wherein X and R₁ are as defined above;
(m) hydroxylating the compound of general structure Va with a suitable hydroxylating agent, wherein X and R₁ are as defined above to give a compound of general structure Va, wherein X represents OR₂ and R₂ represents hydrogen, and wherein R₁ is as defined above;
(o) isomerising the compound of general structure Va to the compound of general structu re VIa, 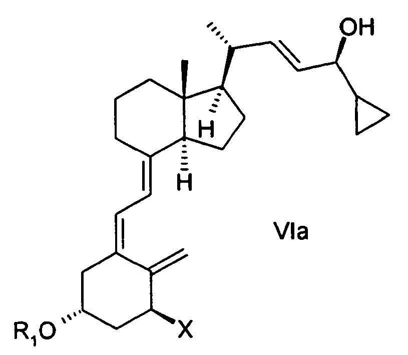 wherein X, R₁ and R₂ are as defined above; and
(p) when R₁ is not hydrogen, removing the hydroxy protecting group R₁ of the compound of general structure VIa to generate calcipotriol or calcipotriol monohydrate.

5. A method for producing calcipotriol or calcipotriol monohydrate comprising steps (j) - (I) of claim 4 and further comprising the steps of:
(q) protecting the C-24 hydroxy group of the compound of general structure Va, 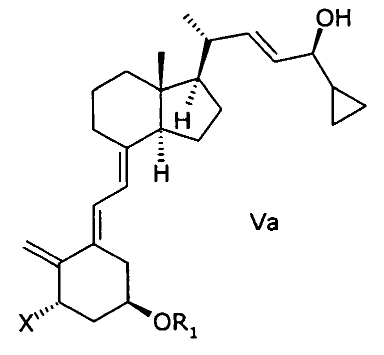 wherein X represents hydrogen, and wherein R₁ represents hydrogen or a hydroxy protecting group, with a hydroxy protecting group;
(r) hydroxylating the C-24 hydroxy protected compound of general structure Va with a suitable hydroxylating agent, wherein X and R₁ are as defined above to give a C-24 hydroxy protected compound of general structure Va, wherein X represents OR₂ and R₂ represents hydrogen, and wherein R₁ is as defined above;
(s) removing the C-24 hydroxy protecting group of the compound of general structure Va;
(t) isomerising the compound of general structure Va to the compound of general structure VIa, 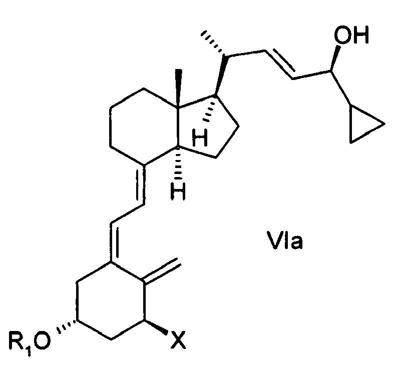 wherein X, R₁ and R₂ are as defined above; and
(u) when R₁ is not hydrogen, removing the hydroxy protecting group R₁ of the compound of general structure VIa to generate calcipotriol or calcipotriol monohydrate.

6. The method according to any one of claims 1-5, wherein the reducing step is with a reducing agent in the presence of a chiral auxiliary.

7. The method according to any one of claims 1-6, wherein the reducing agent is a borane derivative.

8. The method according to claim 6, wherein the reducing agent is *N,N*-diethylaniline-borane, borane-tetrahydrofuran, or borane dimethylsulfide.

9. The method according to any one of claims 1-8, wherein the chiral auxiliary is a chiral 1,2-amino-alcohol.

10. The method according to any one of claims 1-8, wherein the chiral auxiliary is a chiral *cis*-1-amino-2-indanol derivative.

11. The method according to any one of claims 1-8, wherein the chiral auxiliary is (*1S,2R*)-(-)-*cis*-1-amino-2-indanol.

12. The method according to any one of claims 1-11, wherein the inert solvent is toluene, *tert*-butyl methyl ether, tetrahydrofuran, or mixtures thereof.

13. The method according to any one of claims 1-12, wherein the mixture of compounds of general structure IVa and IVb obtained by reducing a compound of general structure III has a molar ratio of IVa:IVb which is at least 56:44.

14. The method according to any one of claims 1-13, wherein the reducing step is carried out at a temperature between 10-20°C.

15. A method for producing a compound of general structure III, 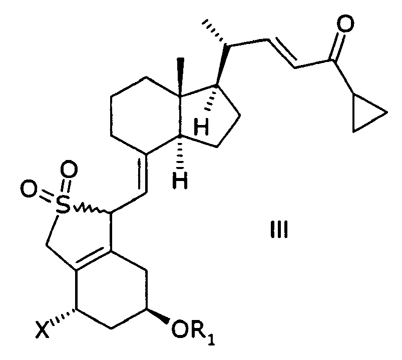 wherein X represents either hydrogen or OR₂,
and wherein R₁ and R₂ may be the same or different and represent hydrogen, or a hydroxy protecting group,
by reacting a compound of general structure VII or VIII, 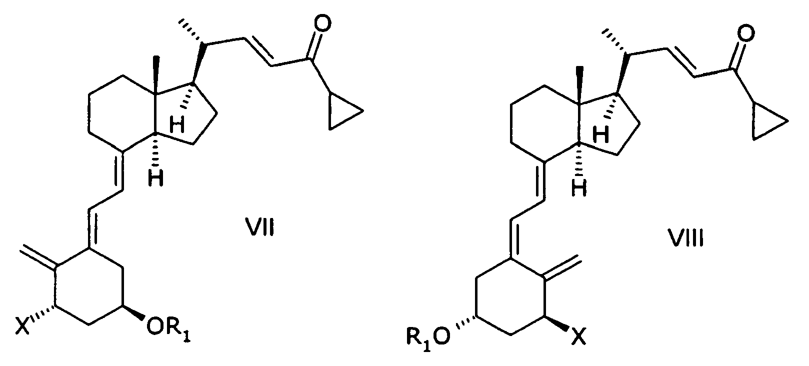 wherein X represents either hydrogen or OR₂,
wherein R₁ and R₂ are as defined above,
with sulphur dioxide.

16. A method according to any one of claims 1-15, wherein the compound of general structure III is the epimer of general structure IIIa

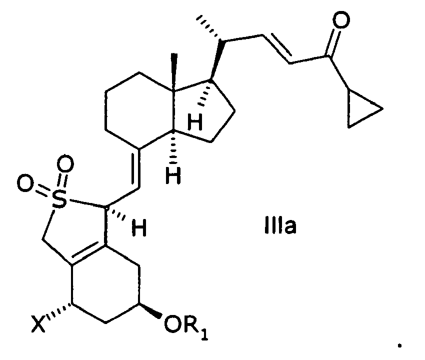

17. A method according to any one of claims 1-15, wherein the compound of general structure III is the epimer of general structure IIIb

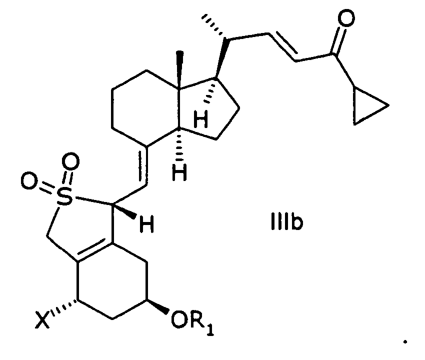

18. A method of reacting the mixture of compounds of general structure IVa and IVb , 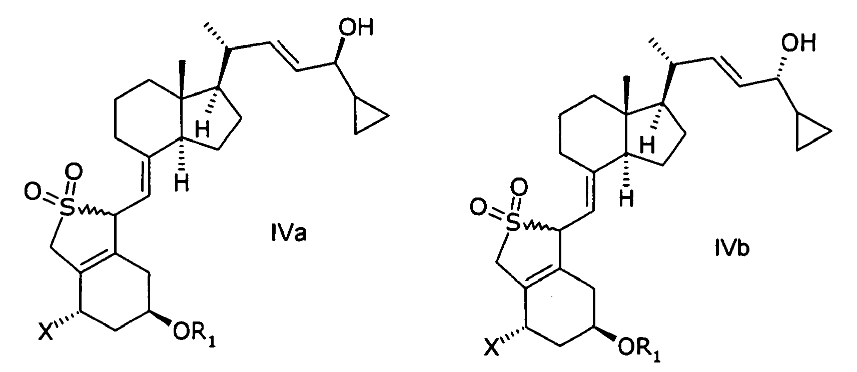 wherein X represents either hydrogen or OR₂,
and wherein R₁ and R₂ may be the same or different and represent hydrogen, or a hydroxy protecting group,
which is enriched with IVa, in the presence of a base to give a mixture of compounds of general structure Va and Vb, which is enriched with Va, 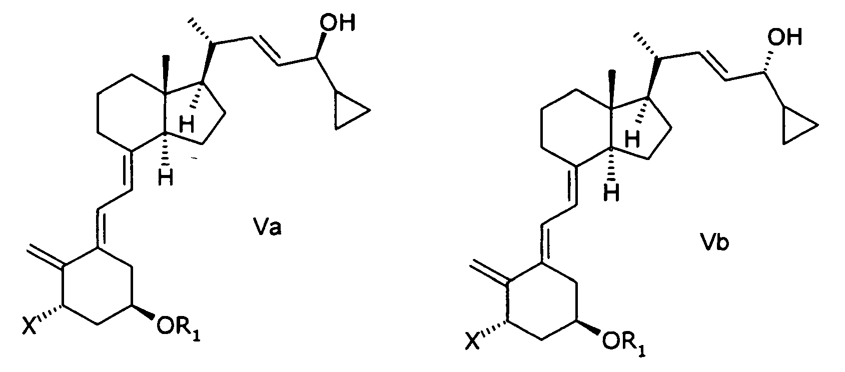 wherein X, R₁, and R₂ are as defined above.

19. A method according to claims 1-3, 15, or 18, wherein X represents OR₂.

20. A method according to any one of claims 1-19, wherein R₁ and/or R₂ represent alkylsilyl.

21. A method according to claim 20, wherein R₁ and/or R₂ represent tert-butyldimethylsilyl.

22. A method for producing calcipotriol {(5Z, 7E, 22E, 24S)-24-cyclopropyl-9,10-secochola-5,7,10(19),22-tetraene-1α-3β-24-triol} or calcipotriol monohydrate comprising the method of any one of claims 1-21.

23. A compound of general structure IIIa or IIIb, or mixtures thereof, 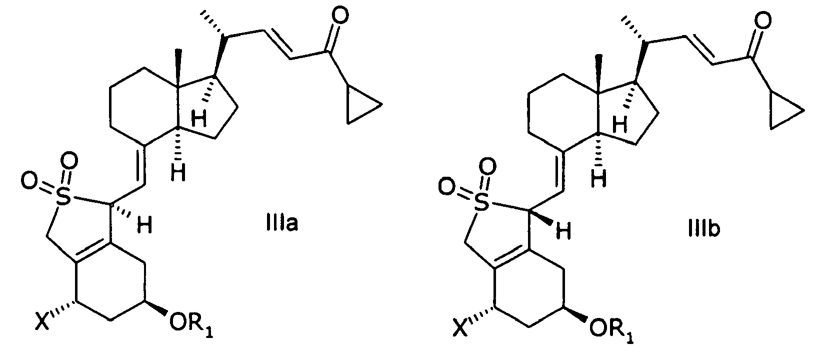 wherein X represents either hydrogen or OR₂,
and wherein R₁ and R₂ may be the same or different and represent hydrogen, or a hydroxy protecting group.

24. A compound of general structure IVaa, IVab, IVba, IVbb, IVb, or mixtures thereof, 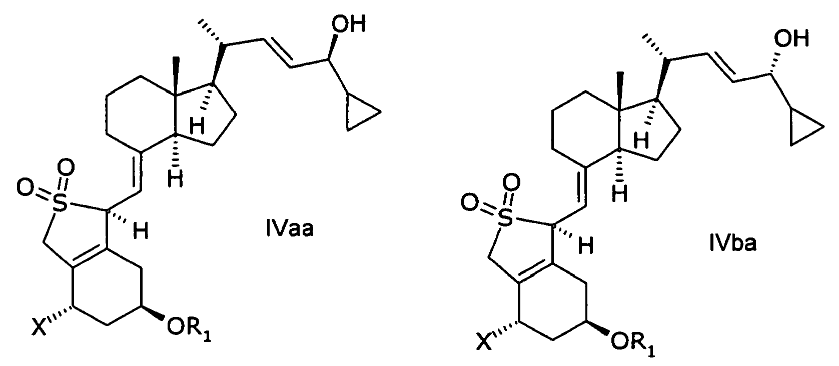 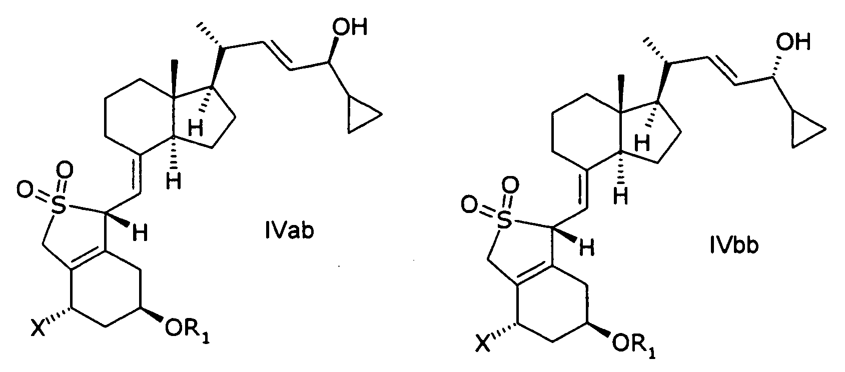 wherein X represents either hydrogen or OR₂,
and wherein R₁ and R₂ may be the same or different and represent hydrogen, or a hydroxy protecting group.

25. A compound according to claim 23 or 24, wherein X represents OR₂.

26. A compound according to any one of claims 23-25, wherein R₁ and R₂ represent alkylsilyl.

27. A compound according to claim 26, wherein R₁ and R₂ represent *tert-*butyldimethylsilyl.

28. A compound according to any one of claims 23-25, wherein R₁ and R₂ represent hydrogen.

29. Use of a compound according to any one of claims 23-28 as an intermediate in the manufacture of calcipotriol or calcipotriol monohydrate.

## Patentansprüche

1. Verfahren zur Reduktion einer Verbindung der allgemeinen Struktur III, 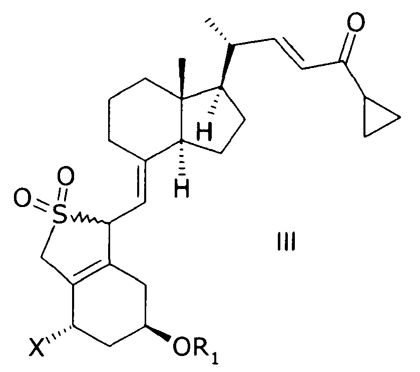 worin X entweder für Wasserstoff oder OR₂ steht,
und worin R₁ und R₂ gleich oder verschieden sein können und für Wasserstoff oder eine Hydroxyschutzgruppe stehen,
in einem inerten Lösungsmittel mit einem chiralen Reduktionsmittel oder mit einem Reduktionsmittel in der Gegenwart eines chiralen Hilfsstoffs,
zu einem Gemisch von Verbindungen der allgemeinen Struktur IVa und IVb, 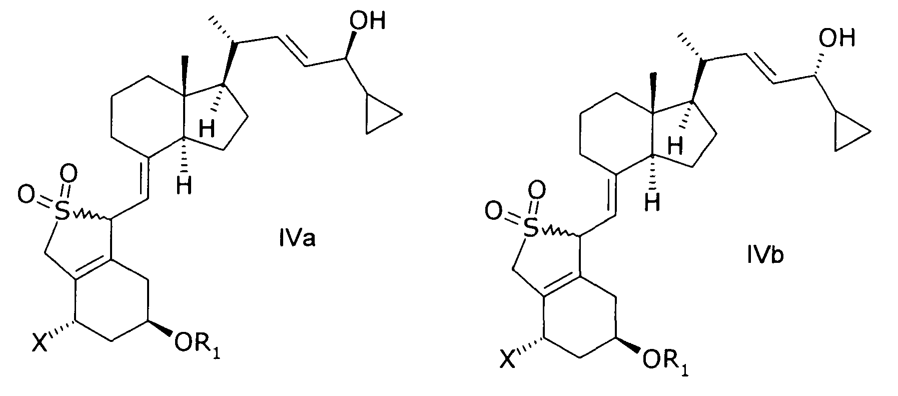 welches mit IVa angereichert ist, worin X, R₁, und R₂ wie obenstehend definiert sind.

2. Verfahren zur Herstellung von Calcipotriol {(5Z,7E,22E,24S)-24-Cyclopropyl-9,10-secochola-5,7,10(19),22-tetraen-1α-3β-24-triol} oder Calcipotriol-Monohydrat, wobei das Verfahren die folgenden Schritte umfasst:
(a) Reduzieren einer Verbindung der allgemeinen Struktur III, 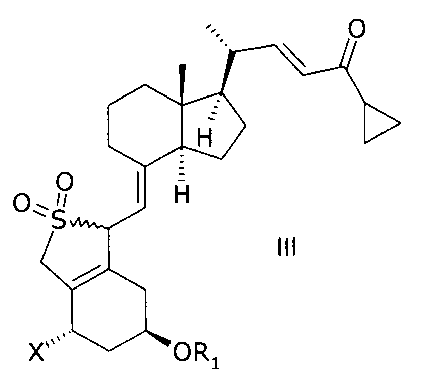 worin X für OR₂ steht,
und worin R₁ und R₂ gleich oder verschieden sein können und für Wasserstoff oder eine Hydroxyschutzgruppe stehen,
in einem inerten Lösungsmittel mit einem chiralen Reduktionsmittel oder mit einem Reduktionsmittel in der Gegenwart eines chiralen Hilfsstoffs,
zu einem Gemisch von Verbindungen der allgemeinen Struktur IVa und IVb, welches mit IVa angereichert ist, 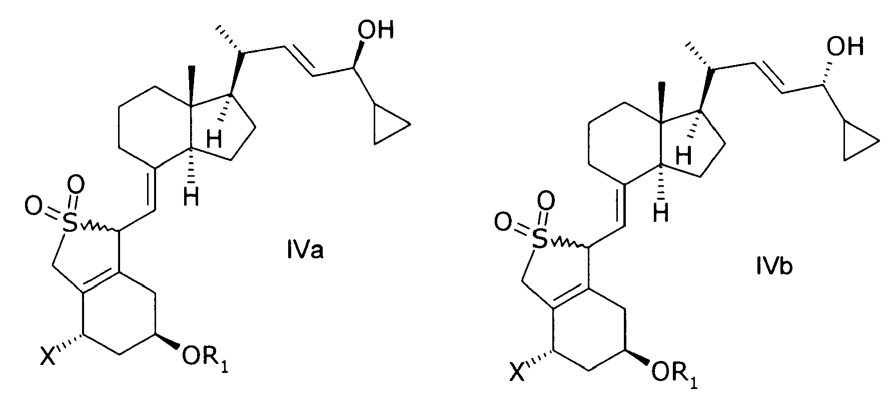 worin X, R₁ und R₂ wie obenstehend definiert sind;
(b) Umsetzen eines Gemisches von Verbindungen der allgemeinen Struktur IVa und IVb, welches mit IVa angereichert ist, in Gegenwart einer Base zu einem Gemisch von Verbindungen der allgemeinen Struktur Va und Vb, welches mit Va angereichert ist, 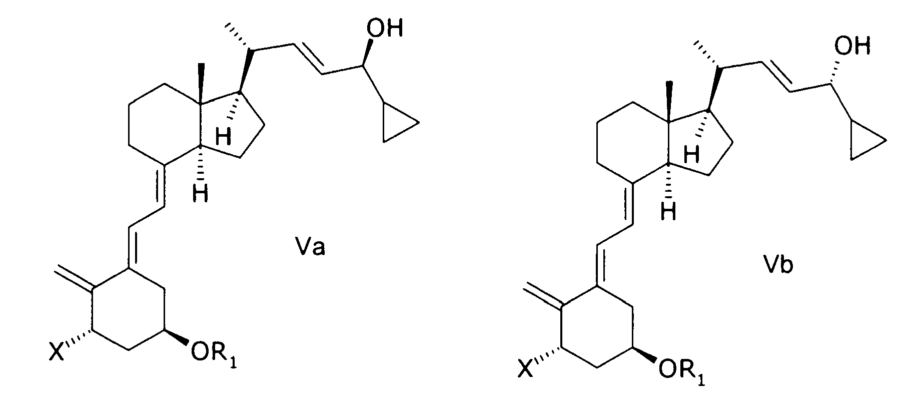 worin X, R₁ und R₂ wie obenstehend definiert sind;
(c) Abtrennen der Verbindung mit der allgemeinen Struktur Va vom Gemisch der Verbindungen der allgemeinen Struktur Va und Vb, welches mit Va angereichert ist,
worin X, R₁ und R₂ wie obenstehend definiert sind;
(d) Isomerisieren der Verbindung der allgemeinen Struktur Va zu einer Verbindung der allgemeinen Struktur VIa, 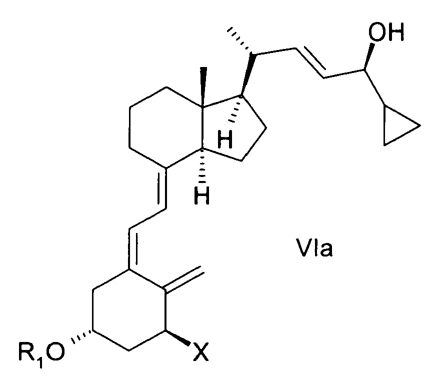 worin X, R₁ und R₂ wie obenstehend definiert sind; und
(e) wenn R₁ und/oder R₂ nicht Wasserstoff sind, Entfernen der Hydroxyschutzgruppe(n) R₁ und/oder R₂ der Verbindung der allgemeinen Struktur VIa, um Calcipotriol oder Calcipotriol-Monohydrat zu bilden.

3. Verfahren zur Herstellung von Calcipotriol oder Calcipotriol-Monohydrat, wobei das Verfahren die Schritte (a) - (b) nach Anspruch 2 und die weiteren folgenden Schritte umfasst:
(f) Isomerisieren des Gemisches von Verbindungen der allgemeinen Struktur Va und Vb, worin X, R₁ und R₂ wie in Anspruch 2 definiert sind, welches mit Va angereichert ist, zu einem Gemisch von Verbindungen der allgemeinen Struktur VIa und Vlb, welches mit VIa angereichert ist, 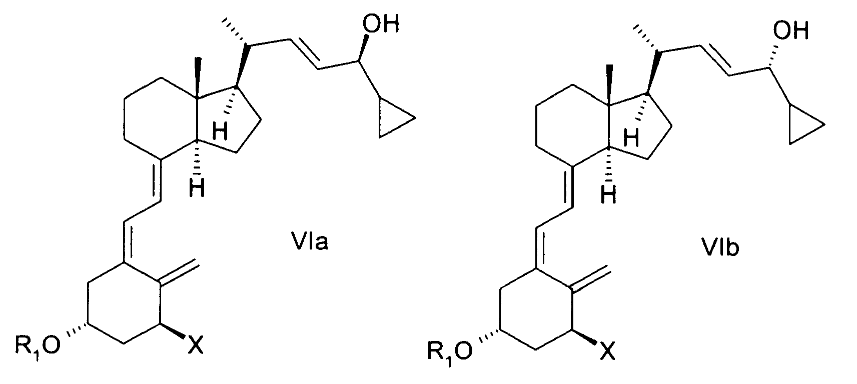 worin X, R₁ und R₂ wie obenstehend definiert sind;
(g) Abtrennen der Verbindung der allgemeinen Struktur VIa vom Gemisch der Verbindungen der allgemeinen Struktur VIa und Vlb, welches mit VIa angereichert ist,
worin X, R₁ und R₂ wie obenstehend definiert sind;
h) wenn R₁ und/oder R₂ nicht Wasserstoff sind, Entfernen der Hydroxyschutzgruppe(n) R₁ und/oder R₂ der Verbindung der allgemeinen Struktur VIa, um Calcipotriol oder Calcipotriol-Monohydrat zu bilden.

4. Verfahren zur Herstellung von Calcipotriol {(5Z,7E,22E,24S)-24-Cyclopropyl-9,10-secochola-5,7,10(19),22-tetraen-1α-3β-24-triol} oder Calcipotriol-Monohydrat, wobei das Verfahren die folgenden Schritte umfasst:
(j) Reduzieren einer Verbindung der allgemeinen Struktur III, 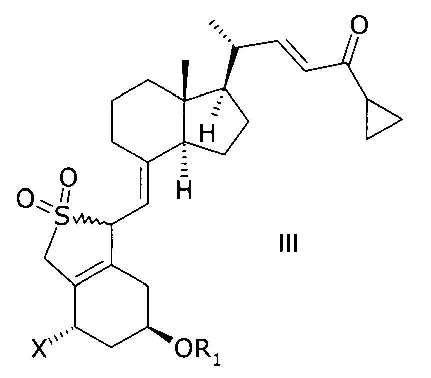 worin X für Wasserstoff steht,
und worin R₁ für Wasserstoff oder eine Hydroxyschutzgruppe steht,
in einem inerten Lösungsmittel mit einem chiralen Reduktionsmittel oder mit einem Reduktionsmittel in der Gegenwart eines chiralen Hilfsstoffs
zu einem Gemisch von Verbindungen der allgemeinen Struktur IVa und IVb, welches mit IVa angereichert ist, 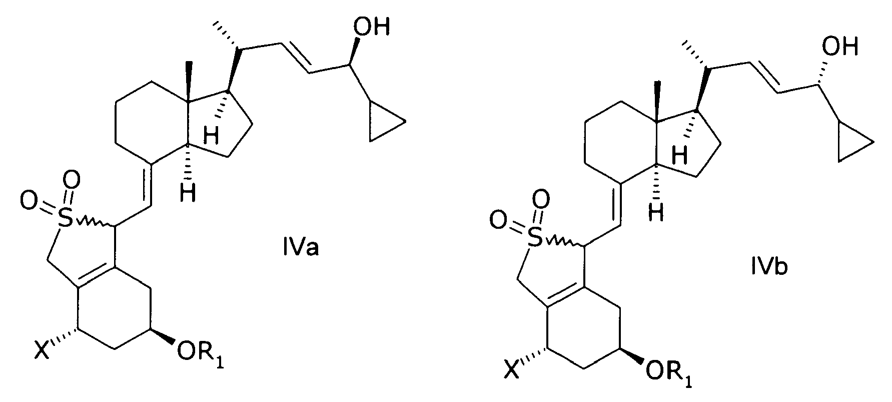 worin X und R₁ wie obenstehend definiert sind;
(k) Umsetzen eines Gemisches von Verbindungen der allgemeinen Struktur IVa und IVb, welches mit IVa angereichert ist, in der Gegenwart einer Base zu einem Gemisch von Verbindungen der allgemeinen Struktur Va und Vb, welches mit Va angereichert ist, 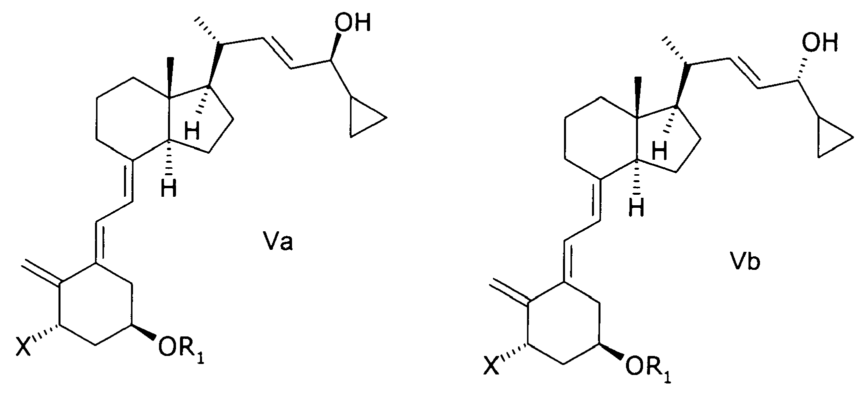 worin X und R₁ wie obenstehend definiert sind;
(I) Abtrennen der Verbindung der allgemeinen Struktur Va von einem Gemisch von Verbindungen der allgemeinen Struktur Va und Vb, welches mit Va angereichert ist, worin X und R₁ wie obenstehend definiert sind;
(m) Hydroxylieren der Verbindung der allgemeinen Struktur Va mit einem geeigneten Hydroxylierungsmittel, worin X und R₁ wie obenstehend definiert sind, zu einer Verbindung der allgemeinen Struktur Va, worin X für OR₂ und R₂ für Wasserstoff steht, und worin R₁ wie obenstehend definiert ist;
(o) Isomerisieren der Verbindung der allgemeinen Struktur Va zu der Verbindung der allgemeinen Struktur VIa, 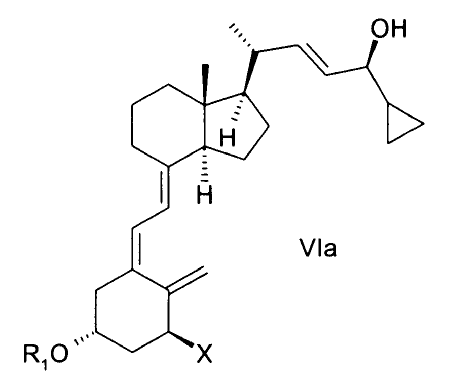 worin X, R₁ und R₂ wie obenstehend definiert sind; und
(p) wenn R₁ nicht Wasserstoff ist, Entfernen der Hydroxyschutzgruppe R₁ der Verbindung der allgemeinen Struktur VIa, um Calcipotriol oder Calcipotriol-Monohydrat zu bilden.

5. Verfahren zur Herstellung von Calcipotriol oder Calcipotriol-Monohydrat, wobei das Verfahren die Schritte (j) - (I) nach Anspruch 4 und die weiteren folgenden Schritte umfasst:
(q) Schützen der C-24-Hydroxygruppe der Verbindung der allgemeinen Struktur Va, 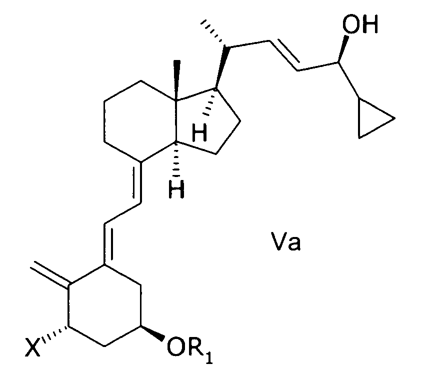 worin X für Wasserstoff steht und worin R₁ für Wasserstoff oder eine Hydroxyschutzgruppe steht, mit einer Hydroxyschutzgruppe;
(r) Hydroxylieren der C-24-Hydroxy-geschützten Verbindung der allgemeinen Struktur Va mit einem geeigneten Hydroxylierungsmittel, worin X und R₁ wie obenstehend definiert sind, zu einer C-24-Hydroxy-geschützten Verbindung der allgemeinen Struktur Va, worin X für OR₂ und R₂ Wasserstoff steht, und worin R₁ wie obenstehend definiert ist;
(s) Entfernen der C-24-Hydroxyschutzgruppe der Verbindung der allgemeinen Struktur Va;
(t) Isomerisieren der Verbindung der allgemeinen Struktur Va zu einer Verbindung der allgemeinen Struktur VIa, 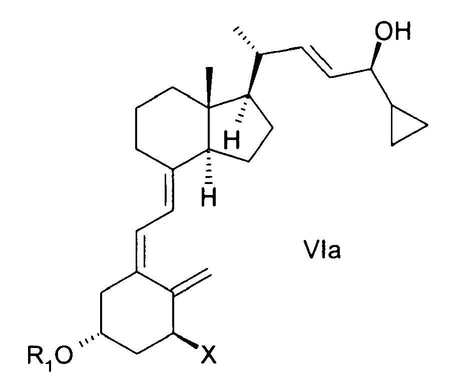 worin X, R₁ und R₂ wie obenstehend definiert sind; und
(u) wenn R₁ nicht Wasserstoff ist, Entfernen der Hydroxyschutzgruppe R₁ der Verbindung der allgemeinen Struktur VIa, um Calcipotriol oder Calcipotriol-Monohydrat zu bilden.

6. Verfahren nach einem der Ansprüche 1-5, wobei der Reduktionsschritt mit einem Reduktionsmittel in der Gegenwart eines chiralen Hilfstoffs durchgeführt wird.

7. Verfahren nach einem der Ansprüche 1-6, wobei das Reduktionsmittel ein Boranderivat ist.

8. Verfahren nach Anspruch 6, wobei das Reduktionsmittel *N,N*-Diethylanilinboran, Borantetrahydrofuran oder Borandimethylsulfid ist.

9. Verfahren nach einem der Ansprüche 1-8, wobei der chirale Hilfsstoff ein chiraler 1,2-Aminoalkohol ist.

10. Verfahren nach einem der Ansprüche 1-8, wobei der chirale Hilfsstoff ein chirales cis-1-Amino-2-indanol-Derivat ist.

11. Verfahren nach einem der Ansprüche 1-8, wobei der chirale Hilfsstoff (*1S,2R*)-(-)-*cis-*1-Amino-2-indanol ist.

12. Verfahren nach einem der Ansprüche 1-11, wobei das inerte Lösungsmittel Toluol, *tert*-Butylmethylether, Tetrahydrofuran oder ein Gemisch davon ist.

13. Verfahren nach einem der Ansprüche 1-12, wobei das Gemisch von Verbindungen der allgemeinen Struktur IVa und IVb, welche man durch Reduktion einer Verbindung der allgemeinen Struktur III erhält, ein molares Verhältnis von IVa : IVb von mindestens 56 : 44 hat.

14. Verfahren nach einem der Ansprüche 1-13, wobei der Reduktionsschritt bei einer Temperatur zwischen 10-20°C ausgeführt wird.

15. Verfahren zur Herstellung einer Verbindung der allgemeinen Struktur III, 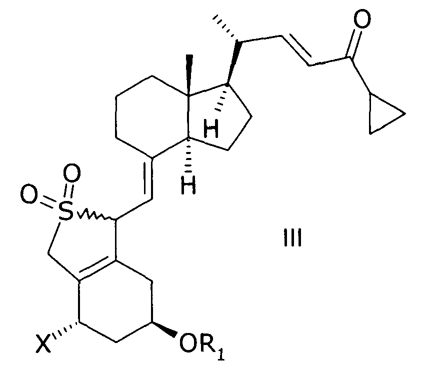 worin X entweder für Wasserstoff oder OR₂ steht,
und worin R₁ und R₂ gleich oder verschieden sein können und für Wasserstoff oder eine Hydroxyschutzgruppe stehen,
durch Umsetzen einer Verbindung der allgemeinen Struktur VII oder VIII, 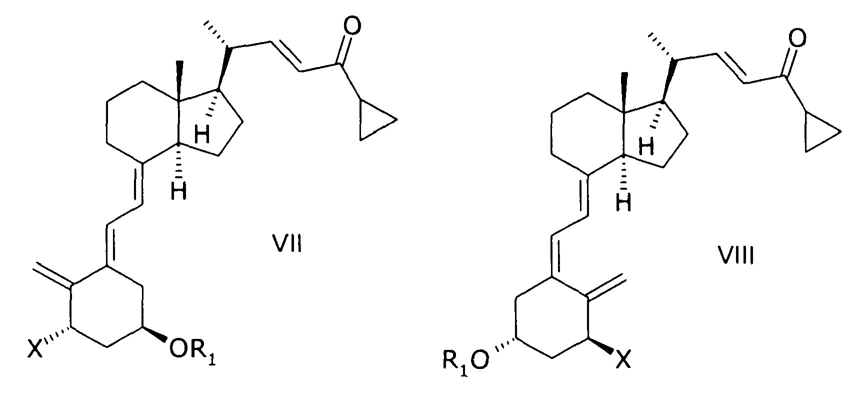 worin X entweder für Wasserstoff oder OR₂ steht,
worin R₁ und R₂ wie obenstehend definiert sind,
mit Schwefeldioxid.

16. Verfahren nach einem der Ansprüche 1-15, wobei die Verbindung der allgemeinen Struktur III das Epimer der allgemeinen Struktur IIIa ist.

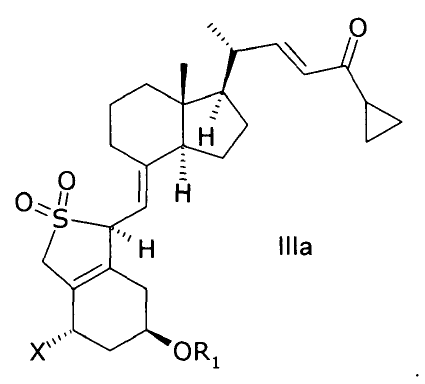

17. Verfahren nach einem der Ansprüche 1-15, wobei die Verbindung der allgemeinen Struktur III das Epimer der allgemeinen Struktur IIIb ist.

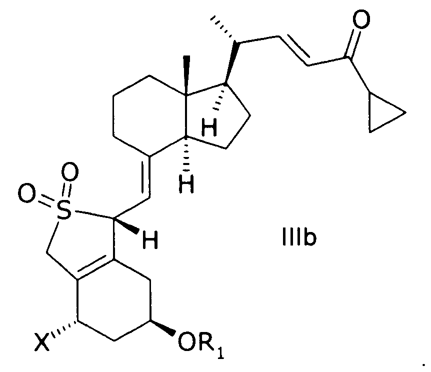

18. Verfahren zur Umsetzung des Gemisches von Verbindungen der allgemeinen Struktur IVa und IVb, 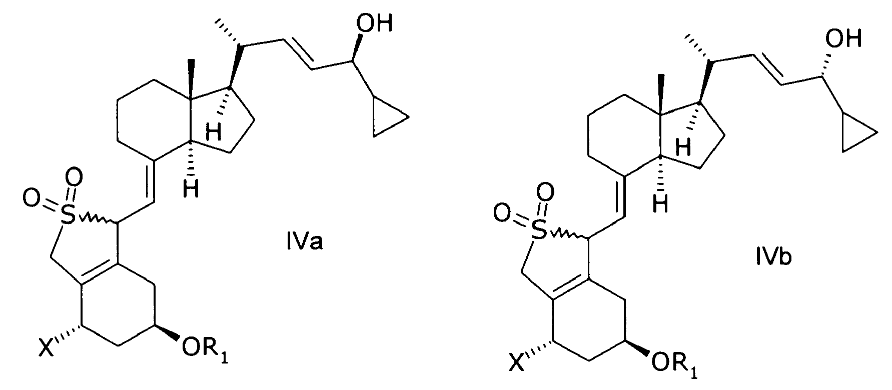 worin X entweder für Wasserstoff oder für OR₂ steht,
und worin R₁ und R₂ gleich oder verschieden sein können und für Wasserstoff oder eine Hydroxyschutzgruppe stehen,
welches mit IVa angereichert ist, in der Gegenwart einer Base zu einem Gemisch von Verbindungen oder allgemeinen Struktur Va und Vb, welches mit Va angereichert ist, 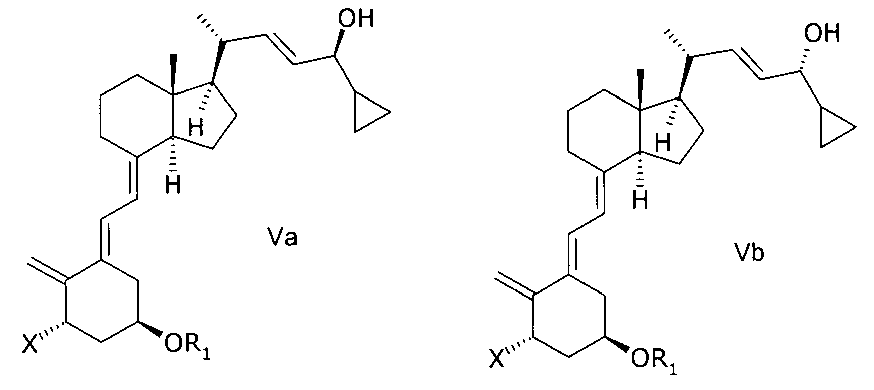 worin X, R₁ und R₂ wie obenstehend definiert sind.

19. Verfahren nach einem der Ansprüche 1-3, 15 oder 18, worin X für OR₂ steht.

20. Verfahren nach einem der Ansprüche 1-19, worin R₁ und/oder R₂ für Alkylsilyl stehen.

21. Verfahren nach einem der Anspruch 20, worin R₁ und/oder R₂ für *tert*-Butyldimethylsilyl stehen.

22. Verfahren zur Herstellung von Calcipotriol {(5Z,7E,22E,24S)-24-Cyclopropyl-9,10-secochola-5,7,10(19),22-tetraen-1α-3β-24-triol} oder Calcipotriol-Monohydrat, wobei das Verfahren ein Verfahren nach einem der Ansprüche 1-21 umfasst.

23. Verbindung der allgemeinen Struktur IIIa oder IIIb oder Gemische hiervon, 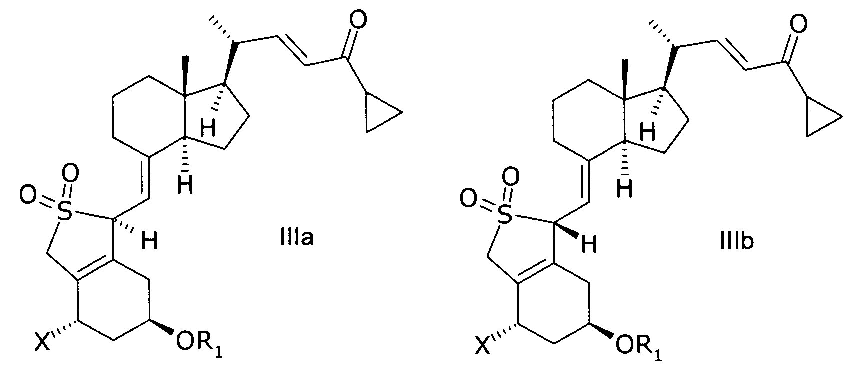 worin X entweder für Wasserstoff oder OR₂ steht,
und worin R₁ und R₂ gleich oder verschieden sein können und für Wasserstoff oder eine Hydroxyschutzgruppe stehen.

24. Verbindung der allgemeinen Struktur IVaa, IVab, IVba, IVbb, IVb oder Gemische davon, 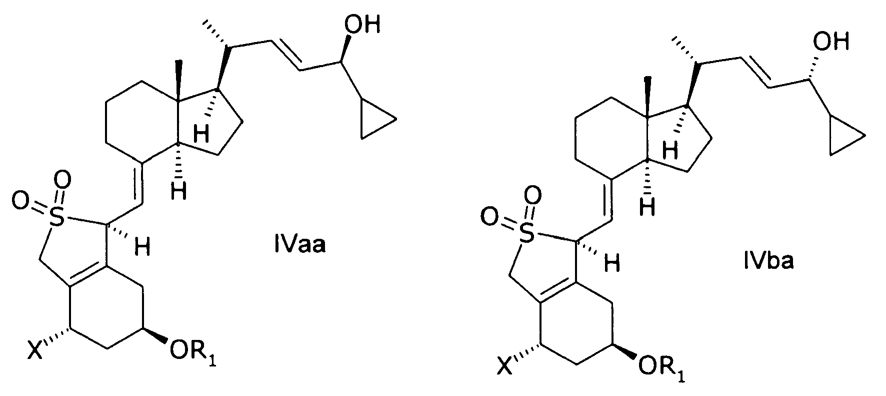 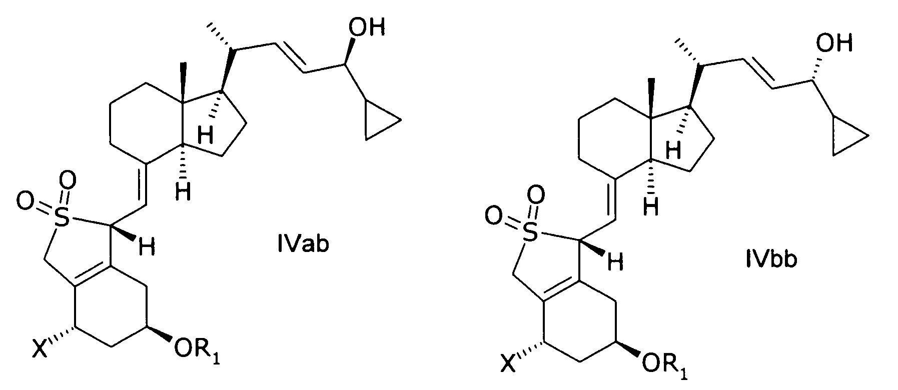 worin X entweder für Wasserstoff oder OR₂ steht,
und worin R₁ und R₂ gleich oder verschieden sein können und für Wasserstoff oder eine Hydroxyschutzgruppe stehen.

25. Verbindung nach Anspruch 23 oder 24, worin X für OR₂ steht.

26. Verbindung nach einem der Ansprüche 23-25, worin R₁ und R₂ für Alkylsilyl stehen.

27. Verbindung nach Anspruch 26, worin R₁ und R₂ für *tert*-Butyldimethylsilyl stehen.

28. Verbindung nach einem der Ansprüche 23-25, worin R₁ und R₂ für Wasserstoff stehen.

29. Verwendung einer Verbindung nach einem der Ansprüche 23-28 als Zwischenprodukt in der Herstellung von Calcipotriol oder Calcipotriol-Monohydrat.

## Revendications

1. Procédé pour réduire un composé de structure générale III, 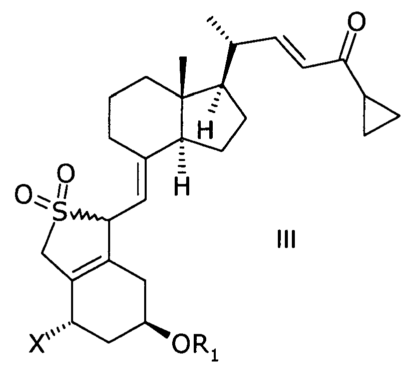 où X représente l'hydrogène ou OR₂,
et où R₁ et R₂ peuvent être identiques ou différents et représentent l'hydrogène ou un groupe protecteur d'hydroxy,
dans un solvant inerte avec un agent réducteur chiral ou avec un agent réducteur en présence d'un auxiliaire chiral,
pour donner un mélange de composés de structure générale IVa et IVb, 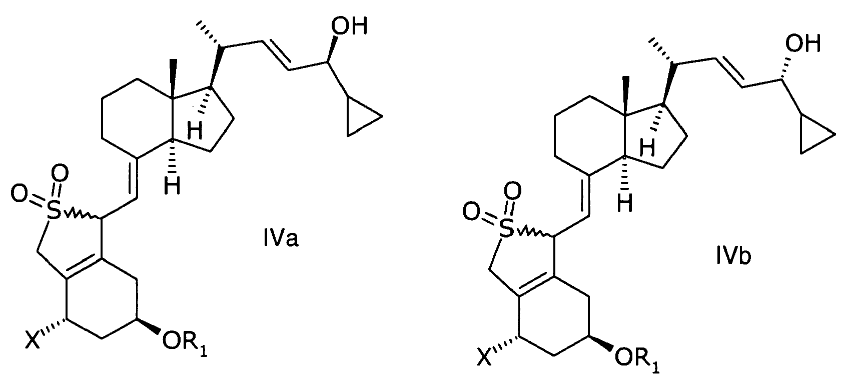 qui est enrichi en IVa, où X, R₁ et R₂ sont définis comme ci-dessus.

2. Procédé pour produire le calcipotriol {(5Z, 7E, 22E, 24S)-24-cyclopropyl-9,10-secochola-5,7,10(19),22-tétraène-1α-3β-24-triol} ou le calcipotriol monohydraté comprenant les étapes de :
(a) réduire un composé de structure générale III, 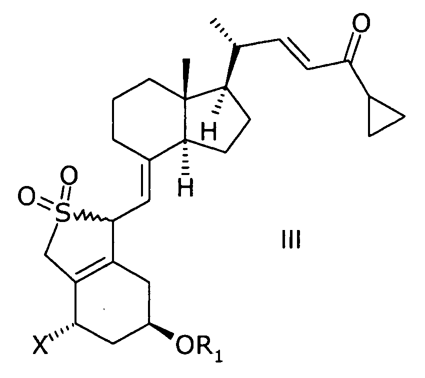 où X représente OR₂,
et où R₁ et R₂ peuvent être identiques ou différents et représentent l'hydrogène ou un groupe protecteur d'hydroxy,
dans un solvant inerte avec un agent réducteur chiral ou avec un agent réducteur en présence d'un auxiliaire chiral,
pour donner un mélange de composés de structure générale IVa et IVb,
qui est enrichi en IVa, 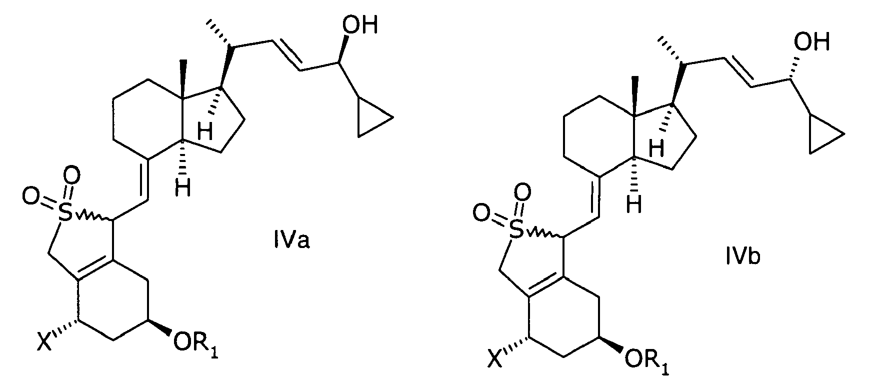 où X, R₁ et R₂ sont définis comme ci-dessus ;
(b) faire réagir le mélange de composés de structure générale IVa et IVb, qui est enrichi en IVa, en présence d'une base pour donner un mélange de composés de structure générale Va et Vb, qui est enrichi en Va, 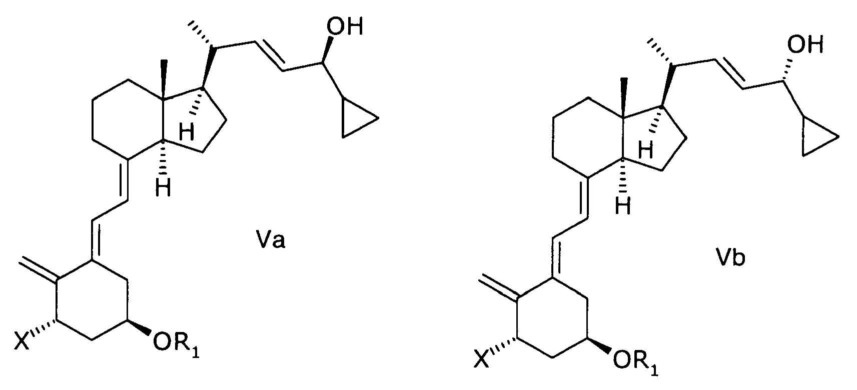 où X, R₁ et R₂ sont définis comme ci-dessus ;
(c) séparer le composé de structure générale Va du mélange de composés de structure générale Va et Vb qui est enrichi en Va, où X, R₁ et R₂ sont définis comme ci-dessus ;
(d) isomériser le composé de structure générale Va en le composé de structure générale VIa 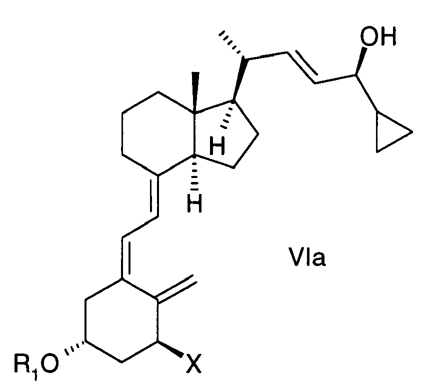 où X, R₁ et R₂ sont définis comme ci-dessus ; et
(e) quand R₁ et/ou R₂ ne sont pas l'hydrogène, retirer le ou les groupes protecteurs d'hydroxy R₁ et/ou R₂ du composé de structure générale VIa pour produire le calcipotriol ou le calcipotriol monohydraté.

3. Procédé pour produire le calcipotriol ou le calcipotriol monohydraté comprenant les étapes (a)-(b) de la revendication 2 et comprenant en outre les étapes de :
(f) isomériser le mélange de composés de structure générale Va et Vb, où X, R₁ et R₂ sont définis comme dans la revendication 2, qui est enrichi en Va, en un mélange de composés de structure générale VIa et VIb, qui est enrichi en VIa, 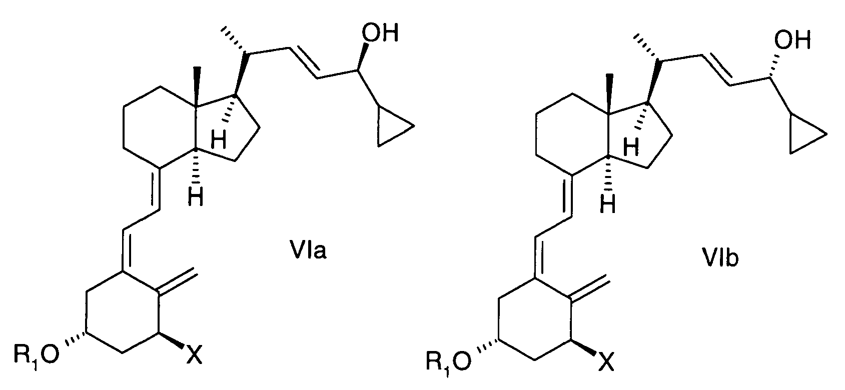 où X, R₁ et R₂ sont définis comme ci-dessus ;
(g) séparer le composé de structure générale VIa du mélange de composés de structure générale VIa et VIb qui est enrichi en VIa, où X, R₁ et R₂ sont définis comme ci-dessus ;
(h) quand R₁ et/ou R₂ ne sont pas l'hydrogène, retirer le ou les groupes protecteurs d'hydroxy R₁ et/ou R₂ du composé de structure générale VIa pour produire le calcipotriol ou le calcipotriol monohydraté.

4. Procédé pour produire le calcipotriol {(5Z, 7E, 22E, 24S)-24-cyclopropyl-9,10-secochola-5,7,10(19),22-tétraène-1α-3β-24-triol} ou le calcipotriol monohydraté comprenant les étapes de :
(j) réduire un composé de structure générale III, 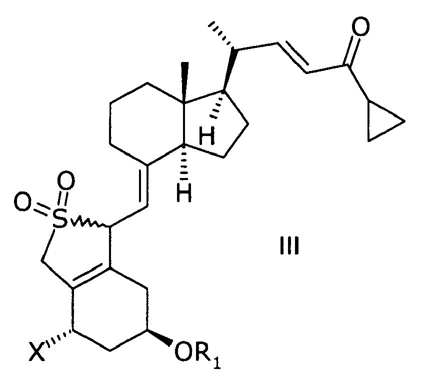 où X représente l'hydrogène,
et où R₁ représente l'hydrogène ou un groupe protecteur d'hydroxy,
dans un solvant inerte avec un agent réducteur chiral ou avec un agent réducteur en présence d'un auxiliaire chiral,
pour donner un mélange de composés de structure générale IVa et IVb,
qui est enrichi en IVa, 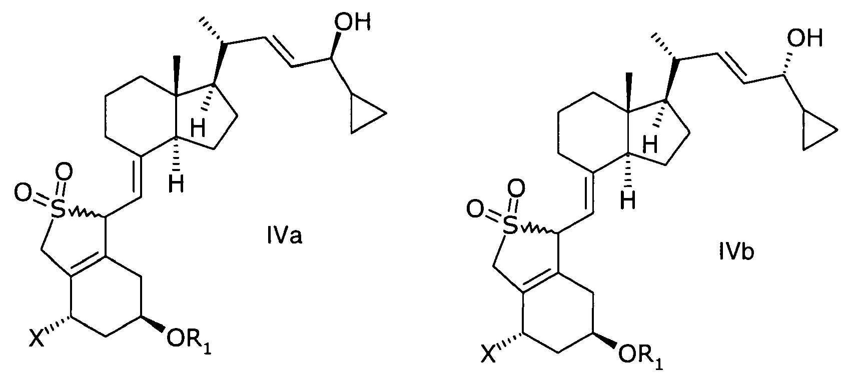 où X et R₁ sont définis comme ci-dessus ;
(k) faire réagir le mélange de composés de structure générale IVa et IVb, qui est enrichi en IVa, en présence d'une base pour donner un mélange de composés de structure générale Va et Vb, qui est enrichi en Va, 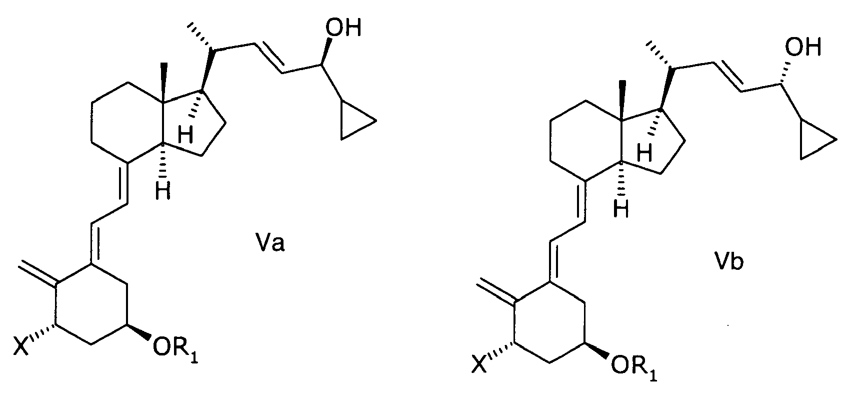 où X et R₁ sont définis comme ci-dessus ;
(l) séparer le composé de structure générale Va du mélange de composés de structure générale Va et Vb qui est enrichi en Va, où X et R₁ sont définis comme ci-dessus ;
(m) hydroxyler le composé de structure générale Va avec un agent hydroxylant approprié, où X et R₁ sont définis comme ci-dessus pour donner un composé de structure générale Va, où X représente OR₂ et R₂ représente l'hydrogène, et où R₁ est défini comme ci-dessus ;
(o) isomériser le composé de structure générale Va en le composé de structure générale VIa, 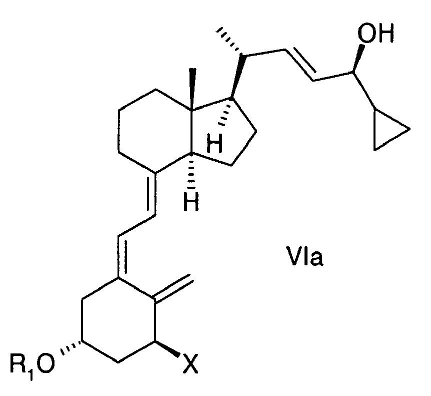 où X, R₁ et R₂ sont définis comme ci-dessus ; et
(p) quand R₁ n'est pas l'hydrogène, retirer le groupe protecteur d'hydroxy R₁ du composé de structure générale VIa pour produire le calcipotriol ou le calcipotriol monohydraté.

5. Procédé pour produire le calcipotriol ou le calcipotriol monohydraté comprenant les étapes (j)-(l) de la revendication 4 et comprenant en outre les étapes de :
(q) protéger le groupe hydroxy C-24 du composé de structure générale Va, 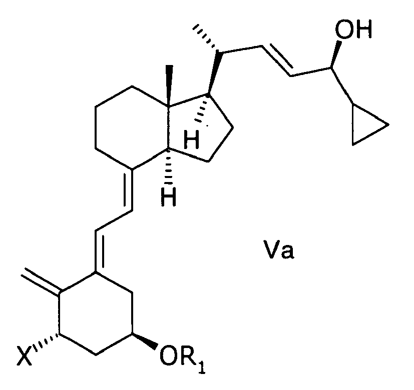 où X représente l'hydrogène et où R₁ représente l'hydrogène ou un groupe protecteur d'hydroxy, avec un groupe protecteur d'hydroxy ;
(r) hydroxyler le composé protégé sur l'hydroxy C-24 de structure générale Va avec un agent hydroxylant approprié, où X et R₁ sont définis comme ci-dessus pour donner un composé protégé sur l'hydroxy C-24 de structure générale Va, où X représente OR₂ et R₂ représente l'hydrogène et où R₁ est défini comme ci-dessus ;
(s) retirer le groupe protecteur d'hydroxy C-24 du composé de structure générale Va ;
(t) isomériser le composé de structure générale Va en le composé de structure générale VIa, 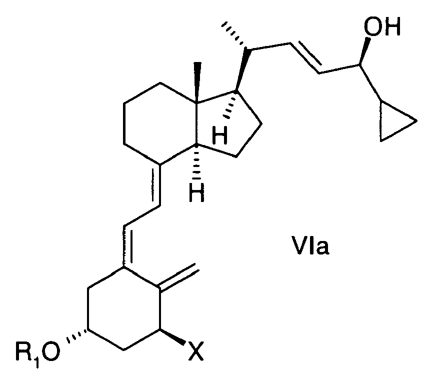 où X, R₁ et R₂ sont définis comme ci-dessus ; et
(u) quand R₁ n'est pas l'hydrogène, retirer le groupe protecteur d'hydroxy R₁ du composé de structure générale VIa pour produire le calcipotriol ou le calcipotriol monohydraté.

6. Procédé selon l'une quelconque des revendications 1-5, où l'étape de réduction est avec un agent réducteur en présence d'un auxiliaire chiral.

7. Procédé selon l'une quelconque des revendications 1-6, où l'agent réducteur est un dérivé de borane.

8. Procédé selon la revendication 6, où l'agent réducteur est le *N,N*-diéthylaniline-borane, le borane-tétrahydrofurane ou le borane diméthylsulfure.

9. Procédé selon l'une quelconque des revendications 1-8, où l'auxiliaire chiral est un 1,2-aminoalcool chiral.

10. Procédé selon l'une quelconque des revendications 1-8, où l'auxiliaire chiral est un dérivé de *cis*-1-amino-2-indanol chiral.

11. Procédé selon l'une quelconque des revendications 1-8, où l'auxiliaire chiral est le (*1S,2R*)-(-)-*cis*-1-amino-2-indanol.

12. Procédé selon l'une quelconque des revendications 1-11, où le solvant inerte est le toluène, le *tert*-butylméthyléther, le tétrahydrofurane ou des mélanges de ceux-ci.

13. Procédé selon l'une quelconque des revendications 1-12, où le mélange de composés de structure générale IVa et IVb obtenu par réduction d'un composé de structure générale III a un rapport molaire de IVa:IVb qui est d'au moins 56:44.

14. Procédé selon l'une quelconque des revendications 1-13, où l'étape de réduction est conduite à une température entre 10-20°C.

15. Procédé pour produire un composé de structure générale III 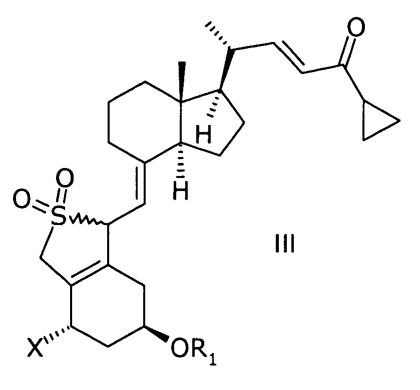 où X représente l'hydrogène ou OR₂,
et où R₁ et R₂ peuvent être identiques ou différents et représentent l'hydrogène, ou un groupe protecteur d'hydroxy, par réaction d'un composé de structure générale VII ou VIII, 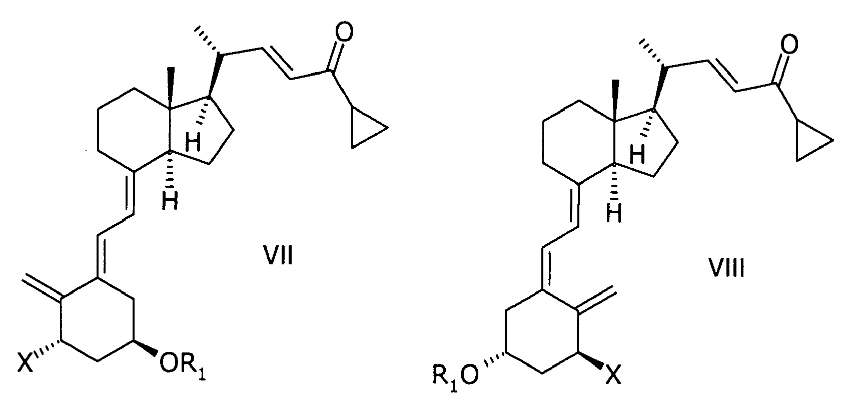 où X représente l'hydrogène ou OR₂,
où R₁ et R₂ sont définis comme ci-dessus,
avec le dioxyde de soufre.

16. Procédé selon l'une quelconque des revendications 1-15, où le composé de structure générale III est l'épimère de structure générale IIIa

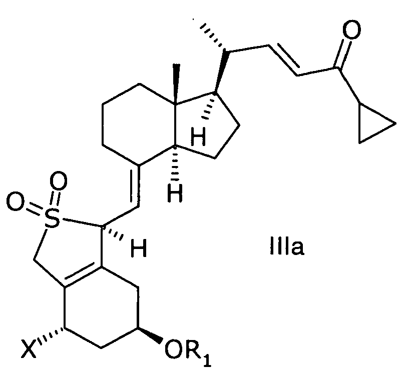

17. Procédé selon l'une quelconque des revendications 1-15, où le composé de structure générale III est l'épimère de structure générale IIIb

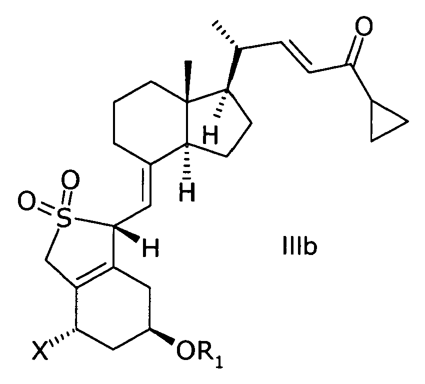

18. Procédé de réaction du mélange de composés de structure générale IVa et IVb 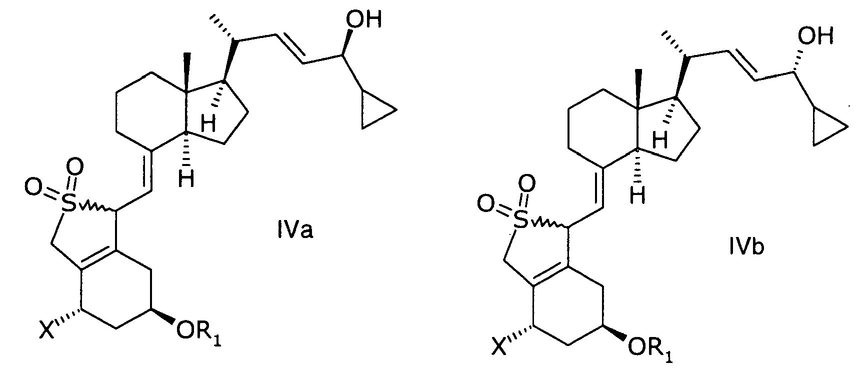 où X représente l'hydrogène ou OR₂,
et où R₁ et R₂ peuvent être identiques ou différents et représentent l'hydrogène, ou un groupe protecteur d'hydroxy,
qui est enrichi en IVa, en présence d'une base pour donner un mélange de composés de structure générale Va et Vb, qui est enrichi en Va, 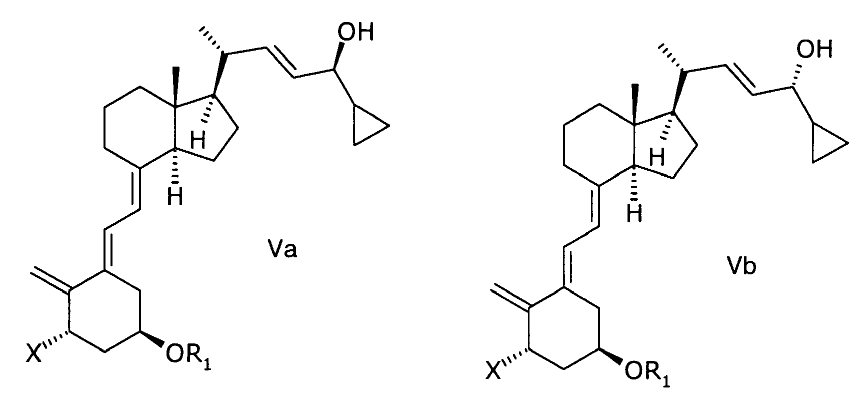 où X, R₁ et R₂ sont définis comme ci-dessus.

19. Procédé selon les revendications 1-3, 15 ou 18, où X représente OR₂.

20. Procédé selon l'une quelconque des revendications 1-19, où R₁ et/ou R₂ représentent alkylsilyle.

21. Procédé selon la revendication 20, où R₁ et/ou R₂ représentent *tert*-butyldiméthylsilyle.

22. Procédé pour produire le calcipotriol {(5Z, 7E, 22E, 24S)-24-cyclopropyl-9,10-secochola-5,7,10(19),22-tétraène-1α-3β-24-triol} ou le calcipotriol monohydraté comprenant le procédé selon l'une quelconque des revendications 1-21.

23. Composé de structure générale IIIa ou IIIb, où mélanges de ceux-ci, 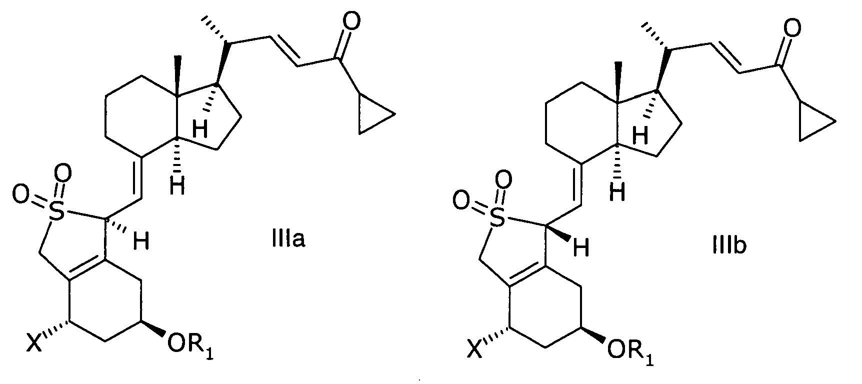 où X représente l'hydrogène ou OR₂,
et où R₁ et R₂ peuvent être identiques ou différents et représentent l'hydrogène, ou un groupe protecteur d'hydroxy.

24. Composé de structure générale IVaa, IVab, IVba, IVbb, IVb, ou mélanges de ceux-ci, 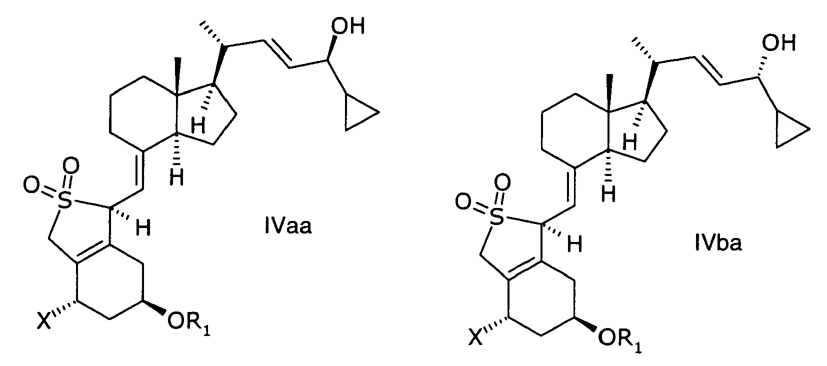 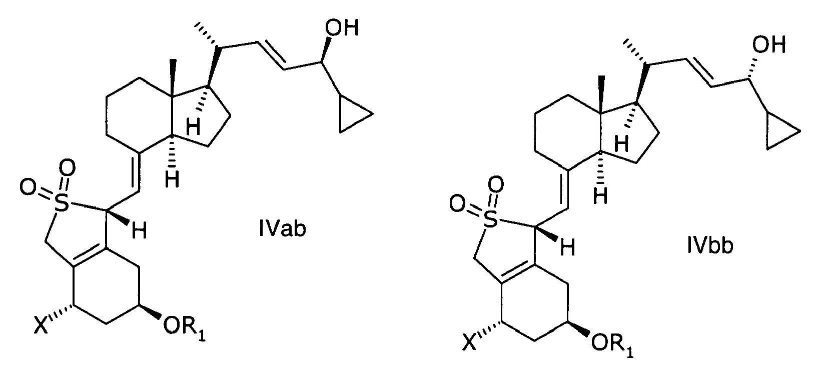 où X représente l'hydrogène ou OR₂,
et où R₁ et R₂ peuvent être identiques ou différents et représentent l'hydrogène, ou un groupe protecteur d'hydroxy.

25. Composé selon la revendication 23 ou 24, où X représente OR₂.

26. Composé selon l'une quelconque des revendications 23-25, où R₁ et R₂ représentent alkylsilyle.

27. Composé selon la revendication 26, où R₁ et R₂ représentent *tert*-butyldiméthylsilyle.

28. Composé selon l'une quelconque des revendications 23-25, où R₁ et R₂ représentent l'hydrogène.

29. Utilisation d'un composé selon l'une quelconque des revendications 23-28 comme intermédiaire dans la production du calcipotriol ou du calcipotriol monohydraté.
